# EUROPEAN PATENT APPLICATION

(11) **EP 4 831 633 A1**
(43) Date of publication of application: **28.10.2026**
(21) Application number: 25172331.8
(22) Date of filing: 24.04.2025
(51) Int. Cl.: G01N 33/533, C12Q 1/48

(54) **BIO-ORTHOGONAL SELF-LABELLING SYSTEM**

(71) Applicant: Forschungsverbund Berlin E.V., 12489 Berlin (DE); Max-Delbrück-Centrum für Molekulare Medizin in der Helmholtz-Gemeinschaft, 13125 Berlin (DE)
(72) Inventor: Broichhagen, Johannes, 12489 Berlin (DE); Birol, Melissa, 12049 Berlin (DE)
(74) Representative: Hertin und Partner Rechts- und Patentanwälte PartG mbB

(57) **Abstract**

The invention relates to a self-labelling bio-orthogonal system, comprising: (A) a protein of interest (POI), (B) two autocatalytically self-splicing amino acid sequences, at least one of said sequences being fused to the POI, wherein the self-splicing of the amino acid sequences is inhibited through steric hindrance, induced by (C) an enzyme located between the two autocatalytically self-splicing amino acid sequences, wherein the enzyme comprises a reactive amino acid (aa_{R}), and is capable of unfolding upon a bio-orthogonal reaction of the reactive amino acid (aa_{R}) with a labelled substrate. The invention further relates accordingly to a method, vector and kit for labelling a protein of interest (POI).

## Description

The invention is in the field of biology, specifically in the field of chemical biology, molecular biology and biochemistry.

The invention relates to a self-labelling bio-orthogonal system comprising: (A) a protein of interest (POI), (B) two autocatalytically self-splicing amino acid sequences, at least one of said sequences being fused to the POI, wherein the self-splicing of the amino acid sequences is inhibited through steric hindrance, induced by (C) an enzyme located between the two autocatalytically self-splicing amino acid sequences, wherein the enzyme comprises a reactive amino acid (aa_{R}), and is capable of destabilizing, and potentially unfolding upon a bio-orthogonal reaction of the reactive amino acid (aa_{R}) with a labelled substrate.

The invention further relates to a method of labelling a protein of interest (POI), comprising providing the self-labelling bio-orthogonal system according to the present invention, contacting the system with a labelled substrate, preferably comprising a fluorescent or radioactive label or dye, thereby inducing a bio-orthogonal labelling reaction of the reactive amino acid (aa_{R}) with the labelled-substrate resulting in (i) the labelling of the aa_{R} (thereby becoming aa_{L}), and (ii) the unfolding of the enzyme (c.), thereby unleashing the steric hindrance and the inhibition of the self-splicing amino acid sequences (b.), and inducing an autocatalytic self-splicing reaction of the system resulting in: (A.) a product comprising the POI and the labelled amino acid (aa_{L}) of the enzyme, and (B.) a product comprising the two (rejoined) self-splicing amino acid sequences and the remaining portion of the enzyme.

The invention further relates to a vector encoding b. and c. of the system according to the present invention, enabling the insertion of one or more sequences encoding the POI (a.). The invention also relates to a kit comprising the system or the vector according to the present invention, and optionally one or more labelled substrate, such as benzylguanine (BG), comprising a fluorescent or radioactive label or dye.

### BACKGROUND OF THE INVENTION

Fluorescence imaging of proteins of interests (POls) requires specific labelling with appropriate chromophores, and careful method consideration is critical ¹⁻³. Fluorescent proteins (FPs), such as StayGold ¹⁵, are inferior to organic fluorophores that display augmented brightness and photostability ^{6,7,16,17}, which makes them attractive in high-resolution and dynamic fluorescence imaging, however, dyes need to be introduced via bio-orthogonal chemistry.

Self-labelling protein tags (SLPs) can be genetically fused to POls and undergo a specific and rapid reaction with synthetic fluorophore-coupled substrates ⁸. Small molecules can be specifically and rapidly reacted with self-labeling protein tags, like the SLPs HaloTag (33 kDa) and SNAP-tag (20 kDa) ⁶, which are comparably large in size to FPs (27 kDa)¹³. As relatively larger tags, however, may disrupt conformations, dynamics and functions of POIs ¹⁸⁻²², the incorporation of unnatural amino acids is an alternative to minimize size-induced perturbations¹⁴. However, applying this approach to live cells is challenging ¹⁶ due to its costs, toxicity, relatively slow labeling kinetics, and putative off-target incorporation. In contrast, other methods, e.g. Lap-tag/LpIA^{W37V 16} or TC-tag/ReAsH ¹⁷, are restricted on the cargo. Taken together, all these points hamper monitoring and tracking dynamical systems.

Modern approaches in fluorescence microscopy, ranging from super-resolution to life-time based imaging, allow scientists to capture diverse dynamics of proteins. However, interrogating native structures, especially within the living cell, remains a major challenge. This is mainly due to traditional methods that utilize large tags (e.g. antibodies, fluorescent and self-labelling proteins). Such tags disturb the respective systems, a fact that has been pointed out many times by researchers across different disciplines.

As such, the problem at hand is the lack of methods combining the superior performance of synthetic fluorophores with nature's toolbox of selective protein labelling. Defining the ideal situation, a fluorescent label should be small, bright, stable, specific, and biologically inert, without the tendency to oligomerize. To date, no system matches all of these criteria. Synthetic fluorophores tick many boxes; however, they are not specific towards a target of interest. Specificity is tractable by genetic engineering, so one has to design a technology that lets the cell do the rest of the work.

### SUMMARY OF THE INVENTION

One of the objectives of the present invention is, in light of the shortcomings of the prior art, the provision of improved or alternative means for specifically labelling a protein of interest (POI), also enabling a small size of the obtained labeled POI that does not disturb protein or intracellular functions.

In light of the prior art, another technical problem underlying the present invention is the provision of improved or alternative means for labelling a POI with a small label using a controllable and straight forward approach that does not impair protein dynamics or intracellular function.

This problem is solved by the features of the independent claims. Preferred embodiments of the present invention are provided by the dependent claims.

The invention therefore relates, in one aspect, to a self-labelling bio-orthogonal system, comprising:
a. a protein of interest (POI),
b. two autocatalytically self-splicing amino acid sequences, at least one of said sequences being fused to the POI, wherein the self-splicing of the amino acid sequences is inhibited through steric hindrance, induced by
c. an enzyme located between the two autocatalytically self-splicing amino acid sequences, wherein the enzyme comprises a reactive amino acid (aaR), and is capable of unfolding upon a bio-orthogonal reaction of the reactive amino acid (aaR) with a labeled substrate.

In embodiments, the self-labelling bio-orthogonal system according to the invention comprises: two autocatalytically self-splicing amino acid sequences, at least one of said sequences being able to be fused to a POI, wherein the self-splicing of the amino acid sequences is inhibited through steric hindrance, induced by an enzyme located between the two autocatalytically self-splicing amino acid sequences, wherein the enzyme comprises a reactive amino acid (aaR), and is capable of unfolding upon a bio-orthogonal reaction of the reactive amino acid (aaR) with a labeled substrate.

In embodiments, the enzyme is endowed with a first part/portion of the two self-splicing of the amino acid sequences and is circularly permutated with the other/second part/portion of the self-splicing amino acid sequences.

In embodiments the self-splicing of the amino acid sequences is inhibited through steric hindrance, preferably by the circular permutation of the enzyme located between the two autocatalytically self-splicing amino acid sequences. Preferably the steric hindrance or sterically induced inhibition of the self-splicing reaction is caused, as the parts of the self-splicing of the amino acid sequences cannot meet due to physical distance caused by the circular permutation of the enzyme.

In other words, in embodiments the self-splicing of the amino acid sequences is inhibited through steric hindrance (a physical distance) induced/caused by the circular permutation of the enzyme located between the two autocatalytically self-splicing amino acid sequences.

Hence, in embodiments the self-splicing of the amino acid sequences (b.) is inhibited through circular permutation induced by (c.) an enzyme located between the two autocatalytically self-splicing amino acid sequences, wherein the enzyme comprises a reactive amino acid (aa_{R}), and is capable of unfolding upon a bio-orthogonal reaction of the reactive amino acid (aa_{R}) with a labeled substrate.

In embodiments, the system may be termed a system "for protein labelling", or "selective protein labelling", or for "self-labelling of a protein", in place of the terminology a "self-labelling bio-orthogonal system".

In embodiments, the bio-orthogonal reaction of the reactive amino acid (aa_{R}) with a labeled (chemically fused) substrate induces (i) the labelling of the aaR (aa_{L}) and (ii) the unfolding of the enzyme (c.), thereby resolving the steric hindrance and inhibition of the two self-splicing amino acid sequences (b.) and inducing an autocatalytic self-splicing reaction of the system.

In embodiments, the enzyme is a suicide enzyme, or a derivative or fragment thereof. In embodiments the enzyme is SNAP or a derivative or fragment thereof. In embodiments, the enzyme comprises the aa_{R} of the *O*⁶-alkylguanine DNA alkyltransferase SNAP, or a derivative thereof, wherein the aa_{R} is Cys145 of SNAP.

In embodiments the enzyme is SNAP or a SNAP-tag. Hence, in preferred embodiments herein 'SNAP' also refers to engineered variants of the suicide enzyme SNAP, such as a SNAP-tag or fragments or derivates of SNAP.

In embodiments, aa_{L} refers to the labelled aa_{R} of the enzyme (resulting from the labelling reaction). In embodiments the enzyme is SNAP and the aa_{R} is amino acid No. 145 (aa145) of SNAP, preferably wherein the labelling results in the label being ligated, connected or attached to/via the aa145 (aa_{L}) of SNAP.

In some embodiments the enzyme is HaloTag. In some embodiments the enzyme is SNAPtag2. In some embodiments the enzyme is CLIP-tag.

In some embodiments the enzyme is a HaloTag and the aa_{R} is an amino acid in the active site of the dehalogenase sequence of the HaloTag. In some embodiments the enzyme is a HaloTag and during the labelling reaction the reactive amino acid aa_{R} of HaloTag binds a reactive chloroalkane linker of a labeled substrate.

In some embodiments the enzyme is a modified SNAP-tag, such as SNAPtag2 or a CLIP-tag, wherein the CLIP-tag has been engineered to react with benzylcytosine (or similar nitrogen containing six-membered heterocyclic) derivatives as labelled substrate.

In general, the self-labelling enzymes according to the present invention preferably undergo (possess) a similar or comparable labelling mechanism. In embodiments, the (self-labelling) enzyme is selected from a SNAP-tag or a CLIP-tag and the reactive amino acid aa_{R} is Cys145. In embodiments the (self-labelling) enzyme is a Halo-tag and the reactive amino acid aa_{R} is Asp106. In embodiments the (self-labelling) is a SNAPtag2 and the reactive amino acid aa_{R} is Cys135.

In embodiments, the induced autocatalytic self-splicing reaction results in:
A. a product comprising the POI and the labeled amino acid (aa_{L}) of the enzyme, and
B. a product comprising at least a portion of the two (optionally rejoined and/or adjacent) self-splicing amino acid sequences and a remaining portion of the enzyme.

In other embodiments, the induced autocatalytic self-splicing reaction results in:
A. a product comprising the POI and the labeled amino acid (aa_{L}) of the enzyme, and
B. a product comprising at least a portion, preferably the major portion, or at least 60%, of the two self-splicing amino acid sequences and a remaining portion, preferably the major portion, or at least 60%, of the enzyme.

In embodiments the induced autocatalytic self-splicing reaction results in product A. and product B., wherein product A. comprises the POI and the labeled amino acid (aa_{L}) of the enzyme, and/or product B. comprises a portion (preferably the major portion, or at least 60%) of the two self-splicing amino acid sequences and a remaining portion, preferably the major portion, or at least 60%, of the enzyme. In embodiments the induced autocatalytic self-splicing reaction results in product A. and product B., wherein product A. comprises the POI, and a certain portion of the enzyme, which comprises at least the labeled amino acid (aa_{L}). In embodiments product A. further comprises a portion/fraction (preferably a minor fraction/portion, or no more than 40%) of the two self-splicing amino acid sequences and/or a fraction/portion (preferably a minor fraction/portion, or no more than 40%) of the enzyme.

In embodiments, the portion of the system (without the POI) comprised within (remaining in) product A. is preferably smaller than 30 kDa, more preferably smaller than 20 kDa, or even smaller than 10 kDa, or even ~5 kDa (without the POI).

In embodiments, the bio-orthogonal reaction of the reactive amino acid (aa_{R}) with a labeled substrate leads to the unfolding of the enzyme and/or system and thereby induces an intramolecular "rearrangement" of the parts of the system, liberating degrees of freedom, such that the two self-splicing amino acid sequences are brough into close proximity or contact, are "rejoined" or in other words are "rearranged" to be positioned in immediate proximity to each other thereby inducing an autocatalytic self-splicing reaction, preferably resulting at least in the products A and B.

In preferred embodiments the present invention provides a method and system that enables chemical labelling with small advantageous labels, such as fluorophores, before transferring the label-containing construct to a POI. The present invention provides preferably a chemo-genetic system that may be provided in embodiments as ready-to-use, transferable to virtually any protein, to virtually any position, e.g., to understand molecular behavior and cellular processes. As such, it promises to optimize state of the art protein labelling and imaging in the life sciences.

The present method preferably enables employing the power of abundant biomolecular machineries to specifically introduce labels, e.g., (synthetic) fluorophores, precisely to otherwise challenging-to-study POls. This present system advantageously enables unprecedented precision in studies targeting protein localization and interactions, as well as real time visualization of protein dynamics in complex tissues. This present system may in embodiments also be applicable in different fields, ranging from single molecule biophysics to cell biology to understanding disease progressions.

The Inventors surprisingly found that the present system and methods preferably enables i) minimizing tag-size, in an ii) controllable manner regarding space and experiment time, in a iii) bio-orthogonal manner, and with an iv) applicability in living cells and enabling iv) the use of synthetic fluorophores. The inventors employed, in one embodiment and by way of example, the engineered suicide enzyme *O*⁶-alkylguanine DNA alkyltransferase termed 'SNAP', which can react with fluorophore-linked benzylguanine (BG) substrates forming a covalent bond on a reactive amino acid (aa_{R}), e.g., cysteine₁₄₅ of SNAP ^{23,24}. In embodiments SNAP refers to the well-known SNAP-tag (only ~20 kDa in size).

One advantage of the present system is that the obtained product A. preferably comprises, due to the self-splicing reaction, a small size, as the portion of the system remaining in product A. (without the POI) is substantially smaller than classical tags used for POl-labelling, such as Halotags (33 kDa), SNAP-tags (20 kDa) or FPs (27 kDa).

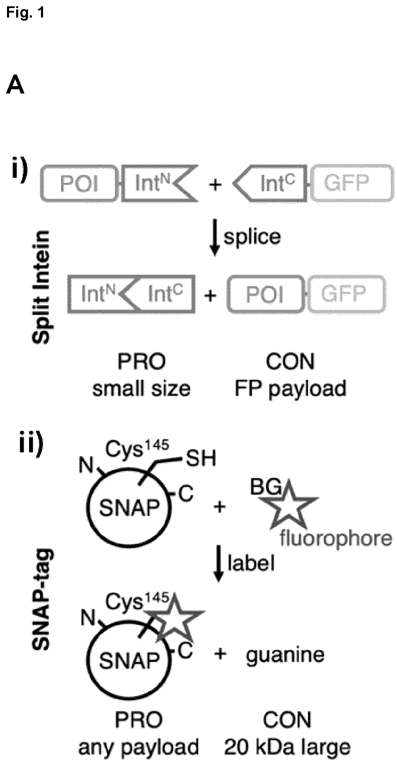

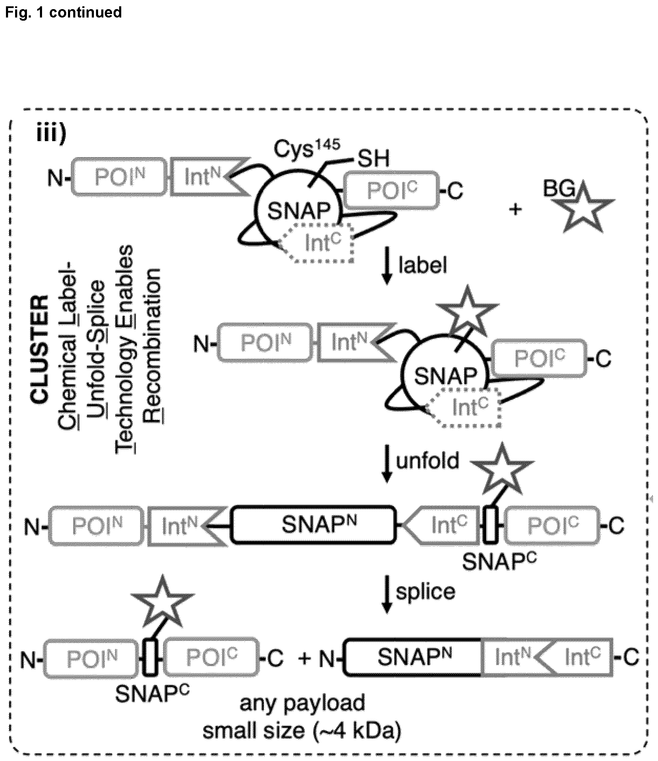

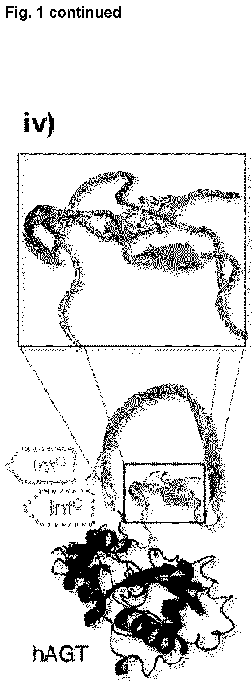

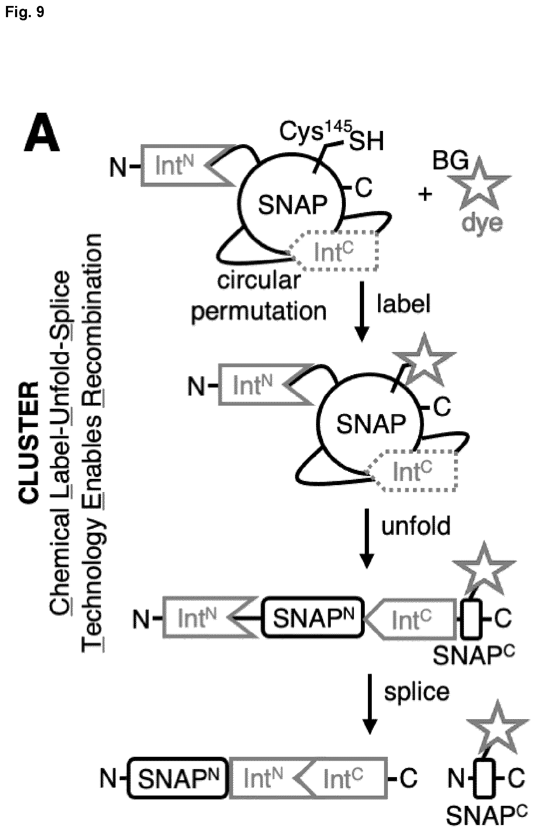

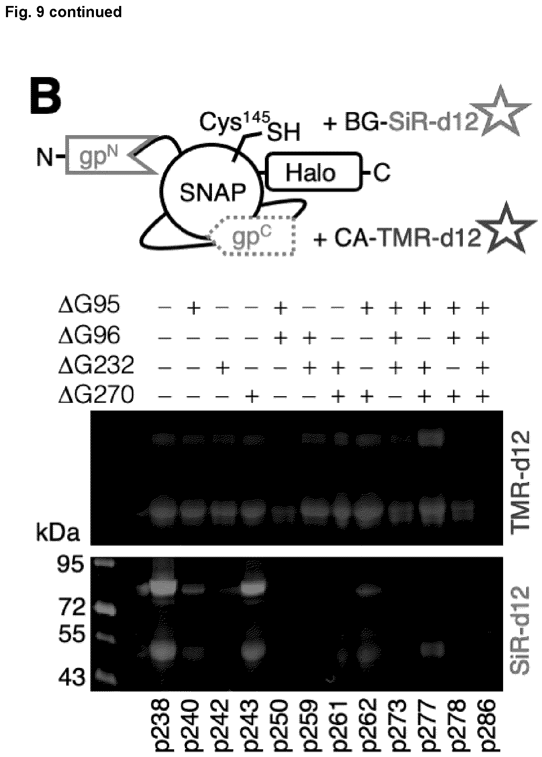

In one non-limiting example described in the following the enzyme SNAP (or SNAP-tag) was circularly permutated between positions 132/133 (thereby also inducing an inhibition through, e.g., steric hinderance, of the self-splicing amino acid sequences) to include autocatalytically self-splicing amino acid sequences, e.g., a split inteinC (gp-41-1C) peptide, upstream of the aa_{R} (e.g., Cys₁₄₅). In said embodiment upon SNAP-labelling, the first portion of the two autocatalytically self-splicing amino acid sequences (e.g., split inteinC gp-41-1C) becomes accessible to its natural interaction partner (the other (second) portion of the two autocatalytically self-splicing amino acid sequences, e.g., gp-41-1N), residing between the N-terminus of the enzyme, e.g., SNAP, and the POI. In said embodiment, when the stoichiometric split parts (of the autocatalytically self-splicing amino acid sequences) meet, they splice the label-, e.g., fluorophore, containing peptide fragment (of the enzyme) onto the POI. An illustrative, non-limiting example is shown in Figs. 1A and 9A/B.

The Inventors could surprisingly show that the present system and method enables, for example, an efficient labelling in living cells, such as, e.g., transmembrane and dynamic intrinsically disordered proteins. Elegantly, in embodiments the present system enables in frame-insertion in between any DNA sequence to include a small (fluorescent) label at any desired protein position (e.g., between or within functional domains) enabling an advantageous strictly stoichiometric reaction. Moreover, in some embodiments where SNAP is used, the exogenous addition of a BG-linked label, e.g., fluorophore, provides exceptional temporal control for labelling.

In embodiments, the two autocatalytically self-splicing amino acid sequences are split intein sequences (InN and IntC), which are preferably silenced/inhibited through steric hindrance (e.g., induced by a circular permutation, e.g., of/with the enzyme) of the IntC (C-terminal intein), and/or the InN (N-terminal intein) portion.

In embodiments, the two autocatalytically self-splicing amino acid sequences are split intein sequences (InN and IntC), wherein the split intein sequences are selected from gp-41-1 (gp-41-1 C and gp-41-1N) and AvaN-NpuC.

In embodiments, the reactive amino acid (aa_{R}) is a cysteine, preferably wherein the enzyme (c.) comprising the aa_{R} is an O⁶-alkylguanine DNA alkyltransferase, SNAP, or a derivative thereof and the aa_{R} is Cy₁₄₅.

In embodiments, the labeled-substrate is selected from benzylguanine (BG), chloropyrimidine (CP), chloroalkane (CA), fluorinated labels, pyrimidine-based, and/or rhodamine-based substrates, or derivatives or functional analogies thereof. In embodiments, the labeled substrate is a benzylguanine (BG) substrate, preferably comprising a label, that is preferably a fluorescent or radioactive label or dye.

In embodiments, the labeled-substrate is selected from benzylguanine (BG), chloropyrimidine (CP), chloroalkane (CA), fluorinated labels, pyrimidine-based, and/or rhodamine-based substrates, or derivatives or functional analogies thereof, comprising a label, preferably a fluorescent or radioactive label or dye. For example, without limitation thereto, additional labeled substrates are known to a skilled person, and by way of example, described in Kühn et al, bioRxiv 2024.08.28.610127; doi: https://doi.org/10.1101/2024.08.28.610127).

In embodiments, the labeled-substrate is selected from orthogonal substrates, such as e.g., pyrimidines, pyrazines, tri-azines, and pyrrolotriazines.

In embodiments, the POI (a.) is divided into a C-terminal and an N-terminal portion, and each portion is fused to one of the two autocatalytically self-splicing amino acid sequences (b.) respectively, and wherein the product (A.) resulting from the autocatalytic self-splicing reaction comprises both the C- and N-terminal portions of the POI.

In embodiments, the system according to the present invention may comprise one or more of the following amino acid sequences disclosed in Table 1.

*Table 1*: Amino acid sequences of exemplary embodiments of the system according to the present invention.

The amino acid sequences are provided as 'full length' sequences comprised within a system, prior to the unfolding and self-splicing of the construct (system) induced by a bio-orthogonal reaction of the reactive amino acid (aaR) with a labeled substrate. The table further discloses exemplary splicing products obtained after the unfolding and splicing reaction. In the table, **bold** typeface represents sequences of the N-terminal or C-terminal sequence of the system/construct; underlined represents the N-terminal portion of the autocatalytically self-splicing amino acid sequences, e.g., split inteinN (Int^{N}); standard typeface represents the enzyme comprising the reactive amino acid (aaR), e.g., SNAP, **bold underlined** typeface represents an reactive amino acid (aaR), e.g., cysteine 145 (C145) of SNAP or aspartic acid 106 (D106), *underlined italic* typeface represents the C-terminal portion of autocatalytically self-splicing amino acid sequences, e.g., split inteinC (Int^{C}), *italic* typeface represents a linker sequence, and ***bold italic*** typeface represents a POI, e.g., for proof of principle experiments a halo tag.

Any one or more of the underlined, bold, italic, or otherwise marked sequences, within the sequences presented below, may be employed in any aspect or embodiment of the invention, independently of the entire sequence presented under the indicated SEQ ID NO. Any of the underlined, bold, italicized, or otherwise marked sequences may therefore represent an embodiment of the invention, and any such sequence is considered disclosed independent of the full sequences presented in the table, in the context of any embodiment or aspect of the invention disclosed herein.

| SEQ ID NO | Amino acid sequence | Description |
|---|---|---|
| 1 | MASWSHPQFEKGADDDDKVPHMTRSGYCLDLKTQVQTPQGM KEISNIQVGDLVLSNTGYNEVLNVFPKSKKKSYKITLEDGKEIICT EEHLFPTQTGEMNISGGLKEGMCLYVKEGGMDKDCEMKRTTL DSPLGKLELSGCEQGLHRIIFLGKGTSAADAVEVPAPAAVLGGP EPLMQATAWLNAYFHQPEAIEEFPVPALHHPVFQQESFTRQVL WKLLKVVKFGEVISYSHLAALAGNPAATAAVKTALSGMMLKKILK IEELDERELIDIEVSGNHLFYANDILTHNGGNPVPILIPCHRVVQG DLDVGGYEGGLAVKEWLLAHEGHRLGKRGRLEVLFQGPKAFLE GSEIGTGFPFDPHYVEVLGERMHYVDVGPRDGTPVLFLHGNPT SSYVWRNIIPHVAPTHRCIAPDLIGMGKSDKPDLGYFFDDHVRF MDAFIEALGLEEVVLVIHDWGSALGFHWAKRNPERVKGIAFME | Full length; **p238: gp41-1N-SNAPf [132GG-133] gp41-1C;** comprising gp41-1 intein; |
| | | wherein POI is halo tag; 74254 Da; |
| | FIRPIPTWDEWPEFARETFQAFRTTDVGRKLIIDQNVFIEGTLPM GVVRPLTEVEMDHYREPFLNPVDREPLWRFPNELPIAGEPANI VALVEEYMDWLHQSPVPKLLFWGTPGVLIPPAEAARLAKSLP NCKAVDIGPGLNLLQEDNPDLIGSEIARWLSTLEISGAPGFSSIS AHHHHHHHHHH | |
| 2 | MASWSHPQFEKGADDDDKVPHMTRSGYTHNGGNPVPILIPCH RVVQGDLDVGGYEGGLAVKEWLLAHEGHRLGKRGRLEVLFQG PKAFLEGSEIGTGFPFDPHYVEVLGERMHYVDVGPRDGTPVLF LHGNPTSSYVWRNIIPHVAPTHRCIAPDLIGMGKSDKPDLGYFF DDHVRFMDAFIEALGLEEVVLVIHDWGSALGFHWAKRNPERV KGIAFMEFIRPIPTWDEWPEFARETFQAFRTTDVGRKLIIDQNVF IEGTLPMGVVRPLTEVEMDHYREPFLNPVDREPLWRFPNELPI AGEPANIVALVEEYMDWLHQSPVPKLLFWGTPGVLIPPAEAAR LAKSLPNCKAVDIGPGLNLLQEDNPDLIGSEIARWLSTLEISGA PGFSSISAHHHHHHHHHH | **Splice product 1** of p238: gp41-1^{N}-SNAPf [132GG-133] gp41-1^{C}; comprising POI (halo tag); |
| | | 45550 Da; |
| 3 | CLDLKTQVQTPQGMKEISNIQVGDLVLSNTGYNEVLNVFPKSKK KSYKITLEDGKEIICTEEHLFPTQTGEMNISGGLKEGMCLYVKEG GMDKDCEMKRTTLDSPLGKLELSGCEQGLHRIIFLGKGTSAADA VEVPAPAAVLGGPEPLMQATAWLNAYFHQPEAIEEFPVPALHH PVFQQESFTRQVLWKLLKVVKFGEVISYSHLAALAGNPAATAAV KTALSGMMLKKILKIEELDERELIDIEVSGNHLFYANDIL | **Splice product 2** of p238: gp41-1^{N}-SNAPf [132GG-133] gp41-1^{C}; |
| | | comprising gp41-1 inteins; |
| | | 45550 Da; |
| 4 | MASWSHPQFEKGADDDDKVPHMGGAEYCLSYDTEVLTVEYG FVPIGEIVDKGIESSVFSIDSNGIVYTQPIAQWHHRGKQEVFEYS LEDGSIIKATKDHKFMTQDGKMLPIDEIFEQELDLLQVKGLPEGG MDKDCEMKRTTLDSPLGKLELSGCEQGLHRIIFLGKGTSAADAV EVPAPAAVLGGPEPLMQATAWLNAYFHQPEAIEEFPVPALHHP VFQQESFTRQVLWKLLKVVKFGEVISYSHLAALAGNPAATAAVK TALSGIKIATRKYLGKQNVYDIGVERDHNFALKNGFIASNCFNKG GNPVPILIPCHRVVQGDLDVGGYEGGLAVKEWLLAHEGHRLGK RGRLEVLFQGPKAFLEGSEIGTGFPFDPHYVEVLGERMHYVDV GPRDGTPVLFLHGNPTSSYVWRNIIPHVAPTHRCIAPDLIGMGK SDKPDLGYFFDDHVRFMDAFIEALGLEEVVLVIHDWGSALGFH WAKRNPERVKGIAFMEFIRPIPTWDEWPEFARETFQAFRTTDV GRKLIIDQNVFIEGTLPMGVVRPLTEVEMDHYREPFLNPVDREP LWRFPNELPIAGEPANIVALVEEYMDWLHQSPVPKLLFWGTPG VLIPPAEAARLAKSLPNCKAVDIGPGLNLLQEDNPDLIGSEIAR WLSTLEISGAPGFSSISAHHHHHHHHHH | Full length; **p239: Ava^{N}-SNAPf [132GG-133]** Npu^{C}, comprising Ava^{N}-Npu^{C} intein; wherein POI is halo tag; |
| | | 75929 Da |
| 5 | MASWSHPQFEKGADDDDKVPHMGGAEYCFNKGGNPVPILIPC HRVVQGDLDVGGYEGGLAVKEWLLAHEGHRLGKRGRLEVLFQ GPKAFLEGSEIGTGFPFDPHYVEVLGERMHYVDVGPRDGTPVL FLHGNPTSSYVWRNIIPHVAPTHRCIAPDLIGMGKSDKPDLGYF FDDHVRFMDAFIEALGLEEVVLVIHDWGSALGFHWAKRNPER VKGIAFMEFIRPIPTWDEWPEFARETFQAFRTTDVGRKLIIDQN | **Splice product 1** of p239: Ava^{N}-SNAPf [132GG-133] Npu^{C}; |
| | | comprising POI (halo tag); |
| | VFIEGTLPMGVVRPLTEVEMDHYREPFLNPVDREPLWRFPNEL PIAGEPANIVALVEEYMDWLHQSPVPKLLFWGTPGVLIPPAEA ARLAKSLPNCKAVDIGPGLNLLQEDNPDLIGSEIARWLSTLEIS GAPGFSSISAHHHHHHHHHH | 45704 Da; |
| 6 | CLSYDTEVLTVEYGFVPIGEIVDKGIESSVFSIDSNGIVYTQPIAQ WHHRGKQEVFEYSLEDGSIIKATKDHKFMTQDGKMLPIDEIFEQ ELDLLQVKGLPEGGMDKDCEMKRTTLDSPLGKLELSGCEQGLH RIIFLGKGTSAADAVEVPAPAAVLGGPEPLMQATAWLNAYFHQP EAIEEFPVPALHHPVFQQESFTRQVLWKLLKVVKFGEVISYSHLA ALAGNPAATAAVKTALSGIKIATRKYLGKQNVYDIGVERDHNFAL KNGFIASN | **Splice product 2** of p239: Ava^{N}-SNAPf [132GG-133] Npu^{C}; |
| | | comprising Ava^{N}-Npu^{C} intein; |
| | | 30243 Da; |
| 7 | MASWSHPQFEKGADDDDKVPHMTRSGYCLDLKTQVQTPQGM KEISNIQVGDLVLSNTGYNEVLNVFPKSKKKSYKITLEDGKEIICT EEHLFPTQTGEMNISGGLKEGMCLYVKEGMDKDCEMKRTTLDS PLGKLELSGCEQGLHRIIFLGKGTSAADAVEVPAPAAVLGGPEP LMQATAWLNAYFHQPEAIEEFPVPALHHPVFQQESFTRQVLWK LLKVVKFGEVISYSHLAALAGNPAATAAVKTALSGMMLKKILKIEE LDERELIDIEVSGNHLFYANDILTHNGNPVPILIPCHRVVQGDLDV GGYEGGLAVKEWLLAHEGHRLGKRGRLEVLFQGPKAFLEGSEI GTGFPFDPHYVEVLGERMHYVDVGPRDGTPVLFLHGNPTSSY VWRNIIPHVAPTHRCIAPDLIGMGKSDKPDLGYFFDDHVRFMD AFIEALGLEEVVLVIHDWGSALGFHWAKRNPERVKGIAFMEFIR PIPTWDEWPEFARETFQAFRTTDVGRKLIIDQNVFIEGTLPMGV VRPLTEVEMDHYREPFLNPVDREPLWRFPNELPIAGEPANIVA LVEEYMDWLHQSPVPKLLFWGTPGVLIPPAEAARLAKSLPNC KAVDIGPGLNLLQEDNPDLIGSEIARWLSTLEISGAPGFSSISAH HHHHHHHHH | Full length; **p262: gp41-1^{N}**-**SNAPf [132GG-133] gp41-1_ΔG95ΔG270;** comprising gp41-1 intein; |
| | | wherein POI is halo tag; |
| 8 | MASWSHPQFEKGADDDDKVPHMTRSGYTHNGNPVPILIPCHR VVQGDLDVGGYEGGLAVKEWLLAHEGHRLGKRGRLEVLFQGP KAFLEGSEIGTGFPFDPHYVEVLGERMHYVDVGPRDGTPVLFL HGNPTSSYVWRNIIPHVAPTHRCIAPDLIGMGKSDKPDLGYFFD DHVRFMDAFIEALGLEEVVLVIHDWGSALGFHWAKRNPERVK GIAFMEFIRPIPTWDEWPEFARETFQAFRTTDVGRKLIIDQNVFI EGTLPMGVVRPLTEVEMDHYREPFLNPVDREPLWRFPNELPIA GEPANIVALVEEYMDWLHQSPVPKLLFWGTPGVLIPPAEAARL AKSLPNCKAVDIGPGLNLLQEDNPDLIGSEIARWLSTLEISGAP GFSSISAHHHHHHHHHH | **Splice product 1** of p262: gp41-1^{N}-SNAPf [132GG-133] gp41-1ΔG95ΔG270; comprising POI (halo tag); 45593 Da |
| 9 | MTRSGYTHNGNPVPILIPCHRVVQGDLDVGGYEGGLAVKEWLL AHEGHRLGKRGRLEVLFQGPKAFLEGSEIGTGFPFDPHYVEVL GERMHYVDVGPRDGTPVLFLHGNPTSSYVWRNIIPHVAPTHRC IAPDLIGMGKSDKPDLGYFFDDHVRFMDAFIEALGLEEVVLVIH DWGSALGFHWAKRNPERVKGIAFMEFIRPIPTWDEWPEFARE TFQAFRTTDVGRKLIIDQNVFIEGTLPMGVVRPLTEVEMDHYRE PFLNPVDREPLWRFPNELPIAGEPANIVALVEEYMDWLHQSPV | **Splice product 1 ΔM1-H21** of p262; Splice product 1 of SEQ ID NO 7 lacking the N-terminal construct |
| | PKLLFWGTPGVLIPPAEAARLAKSLPNCKAVDIGPGLNLLQED NPDLIGSEIARWLSTLEISGAPGFSSISAHHHHHHHHHH | sequence (Δaa1-21) 43214 Da |
| 10 | MASWSHPQFEKGADDDDKVPHMTRSGYCLDLKTQVQTPQGM KEISNIQVGDLVLSNTGYNEVLNVFPKSKKKSYKITLEDGKEIICT EEHLFPTQTGEMNISGGLKEGMCLYVKEGMDKDCEMKRTTLDS PLGKLELSGCEQGLHRIIFLGKGTSAADAVEVPAPAAVLGGPEP LMQATAWLNAYFHQPEAIEEFPVPALHHPVFQQESFTRQVLWK LLKVVKFGEVISYSHLAALAGNPAATAAVKTALSMMLKKILKIEEL DERELIDIEVSGNHLFYANDILTHNGNPVPILIPCHRVVQGDLDV GGYEGGLAVKEWLLAHEGHRLGKRGRLEVLFQGPKAFLEGSEI GTGFPFDPHYVEVLGERMHYVDVGPRDGTPVLFLHGNPTSSY VWRNIIPHVAPTHRCIAPDLIGMGKSDKPDLGYFFDDHVRFMD AFIEALGLEEVVLVIHDWGSALGFHWAKRNPERVKGIAFMEFIR PIPTWDEWPEFARETFQAFRTTDVGRKLIIDQNVFIEGTLPMGV VRPLTEVEMDHYREPFLNPVDREPLWRFPNELPIAGEPANIVA LVEEYMDWLHQSPVPKLLFWGTPGVLIPPAEAARLAKSLPNC KAVDIGPGLNLLQEDNPDLIGSEIARWLSTLEISGAPGFSSISAH HHHHHHHHH | Full length; **p277: gp41-1^{N}**-**SNAPf [132GG-133] gp41-1_ΔG95ΔG232ΔG 270;** |
| | | 73956 Da |
| 11 | MASWSHPQFEKGADDDDKVPHMTRSGYTHNGNPVPILIPCHR VVQGDLDVGGYEGGLAVKEWLLAHEGHRLGKRGRLEVLFQGP KAFLEGSEIGTGFPFDPHYVEVLGERMHYVDVGPRDGTPVLFL HGNPTSSYVWRNIIPHVAPTHRCIAPDLIGMGKSDKPDLGYFFD DHVRFMDAFIEALGLEEVVLVIHDWGSALGFHWAKRNPERVK GIAFMEFIRPIPTWDEWPEFARETFQAFRTTDVGRKLIIDQNVFI EGTLPMGVVRPLTEVEMDHYREPFLNPVDREPLWRFPNELPIA GEPANIVALVEEYMDWLHQSPVPKLLFWGTPGVLIPPAEAARL AKSLPNCKAVDIGPGLNLLQEDNPDLIGSEIARWLSTLEISGAP GFSSISAHHHHHHHHHH | **Splice product 1** of p277: gp41-1^{N}-SNAPf [132GG-133] gp41-1_ΔG95Δ232ΔG2 70; |
| | | comprising POI (halo tag); |
| | | 45565 Da |
| 12 | THNGGNPVPILIPCHRVVQGDLDVGGYEGGLAVKEWLLAHEGH RLGKR | **p262-gp41-1-C-term;** Synthesized C-terminal SNAP-tag peptide (via SPPS); for validation experiment; 5167 Da |
| 13 | MTRSGYCLDLKTQVQTPQGMKEISNIQVGDLVLSNTGYNEVLN VFPKSKKKSYKITLEDGKEIICTEEHLFPTQTGEMNISGGLKEGM CLYVKEGG | **gp41-1^{N} intein** (N-terminal part of qp41-1 intein) |
| 14 | *GMMLKKILKIEELDERELIDIEVSGNHLFYANDILTHNG* | **gp41-1^{C} intein** (C-terminal part of gp41-1 intein) |
| 15 | MTRSGYTHNG | joined splice-product of gp41-1 intein |
| 16 | MGGAEYCLSYDTEVLTVEYGFVPIGEIVDKGIESSVFSIDSNGIV YTQPIAQWHHRGKQEVFEYSLEDGSIIKATKDHKFMTQDGKML PIDEIFEQELDLLQVKGLPEGG | **Ava^{N}**-part (N-terminal part) of Ava^{N}-Npu^{C} intein |
| 17 | *GIKIATRKYLGKQNVYDIGVERDHNFALKNGFIASNCFNKG* | **Npu^{C}**-part (C-terminal part) of Ava^{N}-Npu^{C} intein |
| 18 | MGGAEYCFNKG | joined splice-product of Ava^{N}-Npu^{C} intein |
| 19 | GSEIGTGFPFDPHYVEVLGERMHYVDVGPRDGTPVLFLHGNPT SSYVWRNIIPHVAPTHRCIAPDLIGMGKSDKPDLGYFFDDHVRF MDAFIEALGLEEVVLVIHDWGSALGFHWAKRNPERVKGIAFME FIRPIPTWDEWPEFARETFQAFRTTDVGRKLIIDQNVFIEGTLPM GVVRPLTEVEMDHYREPFLNPVDREPLWRFPNELPIAGEPANI VALVEEYMDWLHQSPVPKLLFWGTPGVLIPPAEAARLAKSLP NCKAVDIGPGLNLLQEDNPDLIGSEIARWLSTLEISG | **Halo-Tag** (e.g., exemplary representing a POI) |
| 20 | *GRLEVLFQGPKAFLE* | **linker sequence** |
| 21 | MDKDCEMKRTTLDSPLGKLELSGCEQGLHRIIFLGKGTSAADAV EVPAPAAVLGGPEPLMQATAWLNAYFHQPEAIEEFPVPALHHP VFQQESFTRQVLWKLLKVVKFGEVISYSHLAALAGNPAATAAVK TALS | **N-terminal SNAP sequence** |
| 22 | GNPVPILIPCHRVVQGDLDVGGYEGGLAVKEWLLAHEGHRLGK R | **C-terminal SNAP sequence** |
| 23 | **MASWSHPQFEKGADDDDKVPH** | **N-terminal** construct sequence |
| 24 | **APGFSSISAHHHHHHHHHH** | **C-terminal** construct sequence |
| 25 | MASWSHPQFEKGADDDDKVPHMTRSGYCLDLKTQVQTPQGM KEISNIQVGDLVLSNTGYNEVLNVFPKSKKKSYKITLEDGKEIICT EEHLFPTQTGEMNISGGLKEGMCLYVKEGGMDKDCEMKRTTL DSPLGKLELSGCEQGLHRIIFLGKGTSAADAVEVPAPAAVLGGP EPLMQATAWLNAYFHQPEAIEEFPVPALHHPVFQQESFTRQVL WKLLKVVKFGEVISYSHLAALAGNPAATAAVKTALSGMMLKKILK IEELDERELIDIEVSGNHLFYANDILTHNGGNPVPILIPCHRVVQG DLDVGGYEGGLAVKEWLLAHEGHRLGKRGRLEVLFQGPKAFLE | N-terminal part of construct **p238: gp41-1N-SNAPf [132GG-133] gp41-1C;** |
| | | comprising gp41-1 intein; |
| | | without POI and C-terminal construct sequence |
| 26 | MASWSHPQFEKGADDDDKVPHMGGAEYCLSYDTEVLTVEYG FVPIGEIVDKGIESSVFSIDSNGIVYTQPIAQWHHRGKQEVFEYS LEDGSIIKATKDHKFMTQDGKMLPIDEIFEQELDLLQVKGLPEGG MDKDCEMKRTTLDSPLGKLELSGCEQGLHRIIFLGKGTSAADAV EVPAPAAVLGGPEPLMQATAWLNAYFHQPEAIEEFPVPALHHP VFQQESFTRQVLWKLLKVVKFGEVISYSHLAALAGNPAATAAVK TALSGIKIATRKYLGKQNVYDIGVERDHNFALKNGFIASNCFNKG GNPVPILIPCHRVVQGDLDVGGYEGGLAVKEWLLAHEGHRLGK RGRLEVLFQGPKAFLE | N-terminal part of construct **p239: Ava^{N}-SNAPf [132GG-133] Npu^{C},** comprising Ava^{N}-Npu^{C} intein; without POI and C-terminal construct sequence |
| 27 | MASWSHPQFEKGADDDDKVPHMTRSGYCLDLKTQVQTPQGM KEISNIQVGDLVLSNTGYNEVLNVFPKSKKKSYKITLEDGKEIICT EEHLFPTQTGEMNISGGLKEGMCLYVKEGMDKDCEMKRTTLDS PLGKLELSGCEQGLHRIIFLGKGTSAADAVEVPAPAAVLGGPEP LMQATAWLNAYFHQPEAIEEFPVPALHHPVFQQESFTRQVLWK LLKVVKFGEVISYSHLAALAGNPAATAAVKTALSGMMLKKILKIEE LDERELIDIEVSGNHLFYANDILTHNGNPVPILIPCHRVVQGDLDV GGYEGGLAVKEWLLAHEGHRLGKRGRLEVLFQGPKAFLE | N-terminal part of construct **p262: gp41-1^{N}-SNAPf [132GG-133] gp41-1_ΔG95ΔG270;** comprising gp41-1 intein; |
| | | without POI and C-terminal construct sequence |
| 28 | MASWSHPQFEKGADDDDKVPHMTRSGYCLDLKTQVQTPQGM KEISNIQVGDLVLSNTGYNEVLNVFPKSKKKSYKITLEDGKEIICT EEHLFPTQTGEMNISGGLKEGMCLYVKEGMDKDCEMKRTTLDS PLGKLELSGCEQGLHRIIFLGKGTSAADAVEVPAPAAVLGGPEP LMQATAWLNAYFHQPEAIEEFPVPALHHPVFQQESFTRQVLWK LLKVVKFGEVISYSHLAALAGNPAATAAVKTALSMMLKKILKIEEL DERELIDIEVSGNHLFYANDILTHNGNPVPILIPCHRVVQGDLDV GGYEGGLAVKEWLLAHEGHRLGKRGRLEVLFQGPKAFLE | N-terminal part of construct **p277: gp41-1^{N}-SNAPf [132GG-133] gp41-1_ΔG95ΔG232ΔG 270;** comprising gp41-1 intein; |
| | | without POI and C-terminal construct sequence |
| 29 | MTRSGYCLDLKTQVQTPQGMKEISNIQVGDLVLSNTGYNEVLN VFPKSKKKSYKITLEDGKEIICTEEHLFPTQTGEMNISGGLKEGM CLYVKEGGMDKDCEMKRTTLDSPLGKLELSGCEQGLHRIIFLGK GTSAADAVEVPAPAAVLGGPEPLMQATAWLNAYFHQPEAIEEF PVPALHHPVFQQESFTRQVLWKLLKVVKFGEVISYSHLAALAGN PAATAAVKTALSGMMLKKILKIEELDERELIDIEVSGNHLFYANDI LTHNGGNPVPILIPCHRVVQGDLDVGGYEGGLAVKEWLLAHEG HRLGKRGRLEVLFQGPKAFLE | Portion of construct **p238: gp41-1N-SNAPf [132GG-133] gp41-1C;** comprising gp41-1 intein; |
| | | without POI and N-/C-terminal construct sequences |
| 30 | MGGAEYCLSYDTEVLTVEYGFVPIGEIVDKGIESSVFSIDSNGIV YTQPIAQWHHRGKQEVFEYSLEDGSIIKATKDHKFMTQDGKML PIDEIFEQELDLLQVKGLPEGGMDKDCEMKRTTLDSPLGKLELS GCEQGLHRIIFLGKGTSAADAVEVPAPAAVLGGPEPLMQATAW LNAYFHQPEAIEEFPVPALHHPVFQQESFTRQVLWKLLKVVKFG EVISYSHLAALAGNPAATAAVKTALSGIKIATRKYLGKQNVYDIGV ERDHNFALKNGFIASNCFNKGGNPVPILIPCHRVVQGDLDVGGY EGGLAVKEWLLAHEGHRLGKRGRLEVLFQGPKAFLE | Portion of construct **p239: Ava^{N}-SNAPf [132GG-133] Npu^{C},** comprising Ava^{N}-Npu^{C} intein; without POI and N-/C-terminal construct sequences |
| 31 | MTRSGYCLDLKTQVQTPQGMKEISNIQVGDLVLSNTGYNEVLN VFPKSKKKSYKITLEDGKEIICTEEHLFPTQTGEMNISGGLKEGM CLYVKEGMDKDCEMKRTTLDSPLGKLELSGCEQGLHRIIFLGKG TSAADAVEVPAPAAVLGGPEPLMQATAWLNAYFHQPEAIEEFP VPALHHPVFQQESFTRQVLWKLLKVVKFGEVISYSHLAALAGNP AATAAVKTALSGMMLKKILKIEELDERELIDIEVSGNHLFYANDILT HNGNPVPILIPCHRVVQGDLDVGGYEGGLAVKEWLLAHEGHRL GKRGRLEVLFQGPKAFLE | Portion of construct **p262: gp41-1^{N}-SNAPf [132GG-133] gp41-1_ΔG95ΔG270;** comprising gp41-1 intein; |
| | | without POI and N-/C-terminal construct sequences |
| 32 | MTRSGYCLDLKTQVQTPQGMKEISNIQVGDLVLSNTGYNEVLN VFPKSKKKSYKITLEDGKEIICTEEHLFPTQTGEMNISGGLKEGM CLYVKEGMDKDCEMKRTTLDSPLGKLELSGCEQGLHRIIFLGKG TSAADAVEVPAPAAVLGGPEPLMQATAWLNAYFHQPEAIEEFP VPALHHPVFQQESFTRQVLWKLLKVVKFGEVISYSHLAALAGNP AATAAVKTALSMMLKKILKIEELDERELIDIEVSGNHLFYANDILTH NGNPVPILIPCHRVVQGDLDVGGYEGGLAVKEWLLAHEGHRLG KRGRLEVLFQGPKAFLE | Portion of construct **p277: gp41-1^{N}-SNAPf [132GG-133] gp41-1_ΔG95ΔG232ΔG** 270; comprising gp41-1 intein; without POI and N-/C-terminal construct sequences |

In embodiments, the system comprises an amino acid sequence selected from SEQ ID NO. 1-32 or a sequence with at least 70% sequence identity thereto, preferably at least 80%, 90%, or 95% identity thereto.

In embodiments, the self-labelling bio-orthogonal system comprises an amino acid sequence selected from SEQ ID NO 25-28, or a sequence with at least 70% sequence identity thereto, and optionally a POI, and/or a C-terminal sequence, such as, e.g., SEQ ID NO 24, or sequence(s) with at least 70%.

In embodiments, the self-labelling bio-orthogonal system comprises an amino acid sequence selected from SEQ ID NO 29-32, or a sequence with at least 70% sequence identity thereto, and optionally a POI, and/or a C-terminal and/or N-terminal sequence, such as, e.g., SEQ ID NO 23 and/or 24, or sequence(s) with at least 70%.

In embodiments the induced autocatalytic self-splicing reaction results in product A. and product B., wherein product A. comprises the POI and the labeled amino acid (aaL) of the enzyme, and/or product B. comprises a portion (preferably the major portion(s), e.g., at least 60%, of the) two self-splicing amino acid sequences and a remaining portion (preferably the major portion, e.g., at least 60%) of the enzyme.

In embodiments of the system according to the invention, product A. comprises an amino acid sequence selected from SEQ ID NO. 2, 5, 8, 9 or 11, or a sequence with at least 70% sequence identity thereto, preferably at least 80%, 90%, or 95% identity thereto.

In embodiments of the system according to the invention, product A. comprises an amino acid sequence selected from SEQ ID NO. 2, 5, 8, 9, 11, 12, 15, 18, 20, 22, 23 or 24, or a sequence with at least 70% sequence identity thereto, preferably at least 80%, 90%, or 95% identity thereto.

In embodiments of the system according to the invention, product B. comprises an amino acid sequence selected from SEQ ID NO. 3, or 6, or a sequence with at least 70% sequence identity thereto, preferably at least 80%, 90%, or 95% identity thereto.

In one aspect the present invention also comprises a method of labelling a protein of interest (POI), comprising
- providing the self-labelling bio-orthogonal system according to the invention,
- contacting the system with a labeled substrate, preferably with benzylguanine (BG) comprising a fluorescent or radioactive label or dye,
- thereby inducing a bio-orthogonal labelling reaction of the reactive amino acid (aa_{R}) with the labeled-substrate resulting in (i) the labelling (optionally through covalent linkage and/or covalent linkage) of the aa_{R} (aa_{L}), and (ii) the unfolding of the enzyme (c.),
- thereby resolving the steric hindrance (preferably induced by circular permutation of the enzyme) and the inhibition of the self-splicing amino acid sequences (b.), and inducing (triggering) an autocatalytic self-splicing reaction of the system resulting in:
   A. a product comprising the POI and the labeled amino acid (aa_{L}) of the enzyme, and
   B. a product comprising at least a portion of the two self-splicing amino acid sequences and a remaining portion of the enzyme.

Hence, in embodiments from the afore method according to the invention product A. and product B. may be obtained, with product A. comprising the POI and the labeled amino acid (aaL) of the enzyme, and/or product B. comprising a portion of the two self-splicing amino acid sequences and a remaining portion of the enzyme.

In embodiments the induced autocatalytic self-splicing reaction results in product A. and product B., wherein product A. comprises the POI and the labeled amino acid (aa_{L}) of the enzyme, and/or product B. comprises a portion (preferably the major portion, or at least 60%) of the two self-splicing amino acid sequences and a remaining portion (preferably the major portion, or at least 60%) of the enzyme.

In embodiments the induced autocatalytic self-splicing reaction results in product A. and product B., wherein product A. comprises the POI, and a certain portion of the enzyme, which comprises at least the labeled amino acid (aaL). In embodiments product A. further comprises a portion/fraction (preferably a minor fraction/portion, or no more than 40%, preferably less) of the two self-splicing amino acid sequences and/or a portion/fraction (preferably a minor fraction/portion, or no more than 40%, preferably less) of the enzyme. An advantage of such embodiments, for example, is that not only the labelling reaction of the POI constitutes a controllable and straight forward approach that does not impair protein dynamics or intracellular function, but that, due to the small size of the obtained product A., functionality of the POI and intracellular mechanisms are not disturbed or impaired.

In embodiments of the method according to the invention, the two autocatalytically self-splicing amino acid sequences (b.) are split intein sequences (Int^{N} and Int^{C}), which are preferably silenced/inhibited through steric hindrance of the Int^{C} and/or the Int^{N} portion (preferably through circular permutation of the enzyme). In embodiments the reactive amino acid (aa_{R}) is a cysteine, preferably wherein the enzyme (c.) comprising the aaR is the O⁶-alkylguanine DNA alkyltransferase SNAP and the aaR is Cys₁₄₅.

In embodiments the enzyme, preferably SNAP, is circularly permutated to include the C-terminal portion of an autocatalytically self-splicing amino acid sequence, preferably a split intein^{C} (Int^{C}) peptide, upstream of a reactive amino acid (aa_{R}) of the enzyme, preferably Cys¹⁴⁵ of SNAP.

In embodiments, the self-splicing of the amino acid sequences is inhibited through circular permutation induced by an enzyme located between the two autocatalytically self-splicing amino acid sequences. Therefore, in embodiments during the bio-orthogonal labelling reaction of the reactive amino acid (aa_{R}) with the labeled-substrate (i), the aaR (aa_{L}) is labelled, and (ii) the unfolding of the enzyme resolves the circular permutation and thereby the inhibition of the self-splicing amino acid sequences, such that an autocatalytic self-splicing reaction is induced and products A. and B. are obtained.

In embodiments upon unfolding of the permutated enzyme, the C-term of the autocatalytically self-splicing amino acid sequences, e.g., Int^{C}, such as gp41-1C or NpuC, will become accessible to its natural interaction partner, namely the N-term of the autocatalytically self-splicing amino acid sequences, e.g., Int^{N}, such as gp41-1 N or AvaN, that resides between the N-terminus (N-terminal part) of the enzyme, e.g., SNAP, and the POI.

In embodiments of the system according to the invention, product A. comprises an amino acid sequence selected from SEQ ID NO. 2, 5, 8, 9 or 11, or a sequence with at least 70% sequence identity thereto, preferably at least 80%, 90%, or 95% identity thereto.

In embodiments of the system according to the invention, product A. comprises an amino acid sequence selected from SEQ ID NO. 2, 5, 8, 9, 11, 12, 15, 18, 20, 22 or 24, or a sequence with at least 70% sequence identity thereto, preferably at least 80%, 90%, or 95% identity thereto.

In embodiments of the system according to the invention, product B. comprises an amino acid sequence selected from SEQ ID NO. 3, or 6, or a sequence with at least 70% sequence identity thereto, preferably at least 80%, 90%, or 95% identity thereto.

In embodiments, the system comprises an amino acid sequence selected from SEQ ID NO. 1-32 or a sequence with at least 70% sequence identity thereto.

In embodiments, the system comprises an amino acid sequence selected from SEQ ID NO 25-28, or a sequence with at least 70% sequence identity thereto, and optionally a POI and/or a C-terminal sequence, such as, e.g., SEQ ID NO 24.

In embodiments, the system comprises an amino acid sequence selected from SEQ ID NO 29-32, or a sequence with at least 70% sequence identity thereto, and optionally a POI and/or a C-terminal and/or N-terminal sequence, such as, e.g., SEQ ID NO 23 and/or 24.

In one aspect the present invention also comprises a vector encoding b. and c. of the system according to the invention, and at least one multiple cloning site (MCS) enabling the insertion of one or more sequences encoding the POI (a.).

In other embodiments, the vector is assembled through alternative cloning techniques, such as, e.g. Gibson assembly.

In embodiments, the vector according to the invention comprises a nucleic acid sequence encoding one or more of the sequences according to SEQ ID NO. 1-32, or a sequence with at least 70% sequence identity thereto, preferably at least 80%, 90%, or 95% identity thereto.

In embodiments, the vector according to the invention comprises a nucleic acid sequence encoding one or more of the sequences according to SEQ ID NO 25-28, or a sequence with at least 70% sequence identity thereto, and optionally a POI and/or a C-terminal sequence, such as, e.g., SEQ ID NO 24, or a sequence with at least 70% sequence identity thereto.

In embodiments, the vector according to the invention comprises a nucleic acid sequence encoding one or more of the sequences according to SEQ ID NO 29-32, or a sequence with at least 70% sequence identity thereto, and optionally a POI and/or a C-terminal and/or N-terminal sequence, such as, e.g., SEQ ID NO 23 and/or 24, or sequence(s) with at least 70% sequence identity thereto.

In one aspect the present invention also comprises a kit comprising the system according to the present invention and/or or the vector according to the present invention, and optionally one or more labeled substrate, preferably benzylguanine (BG), chloropyrimidine (CP), chloroalkane (CA), fluorinated labels, pyrimidine-based, and/or rhodamine-based substrates, or derivatives thereof, comprising a label, preferably a fluorescent label or dye.

The present system, methods and kits are considered to be disclosed with respect to each and every other aspect of the disclosure, such that features characterizing the system may be employed to characterize the methods, or kits and vice-versa. The various aspects of the invention are unified by, benefit from, are based on and/or are linked by the common and surprising finding of the improved labeling of proteins of interest (POls) achieved by the self-labelling bio-orthogonal system described herein.

### DETAILED DESCRIPTION OF THE INVENTION

All cited documents of the patent and non-patent literature are hereby incorporated by reference in their entirety.

The present invention is directed to a self-labeling bio-orthogonal system and methods relating thereto.

In general, "bio-orthogonal" chemistry refers to a class of highly selective chemical reactions that occur within living systems without interfering with native biochemical processes. Typically, these reactions involve functional groups absent from biological environments, ensuring exclusive reactivity between designated partners. To be bio-orthogonal, a reaction must exhibit high selectivity, rapid kinetics under physiological conditions, inertness to biological molecules, and biocompatibility. Common bio-orthogonal reactions include but are not limited to native chemical ligation, Staudinger ligation, copper-free azide-alkyne cycloaddition (SPAAC), tetrazine ligation, oxime and hydrazone ligations, and emerging strategies such as quadricyclane ligation. These reactions facilitate precise biomolecular modifications, e.g., crucial for imaging, drug delivery, metabolic labeling, and proteomics applications.

In the context of the present invention, the term "protein of interest" (hereinafter "POI") refers to any protein expressed in a living cell that is capable of being labeled. In certain embodiments, the method disclosed herein comprises the genetic fusion of a fluorescent peptide to the POI, thereby enabling specific labeling while minimizing disruption to the native biological system. In additional embodiments, the term POI further includes transmembrane proteins and intrinsically disordered proteins.

The terms "autocatalytic" or "autocatalysis" refer to a chemical or biological process in which a product of a reaction catalyzes the same reaction, thereby enhancing its own formation. In a broader sense, a set of chemical reactions can be collectively autocatalytic if the products of some reactions catalyze others within the system, ensuring self-sustainability given an input of energy and precursor molecules. In biological systems, autocatalysis describes a self-perpetuating or self-amplifying process where a molecule facilitates its own production or modification. This principle applies to self-splicing, which preferably comprises in the context of the present invention a form of protein/peptide processing in which a protein/peptide molecule catalyzes its excision without the need for additional enzymes. Unlike traditional autocatalysis, where a molecule generates more of itself, self-splicing is autocatalytic in that the nucleic acid or amino acid sequence promotes a structural modification, its own excision, through intrinsic catalytic activity. The autocatalytic nature of self-splicing is characterized by the presence of an internal catalyst, as the molecule itself mediates the cleavage and ligation reactions. The absence of external enzymes distinguishes self-splicing from other RNA- or protein-processing events and underscores its evolutionary and functional significance. While the reaction does not amplify the molecule in quantity, it is self-sustaining within the context of the catalytic event. Thus, self-splicing exemplifies an autocatalytic mechanism that operates through structural rearrangement rather than replication.

In general, in the context of nucleic acids "self-splicing" may refer to an RNA-mediated catalytic process in which specific introns excise themselves from a precursor RNA molecule without requiring proteins or external enzymes. In the context of the present invention, "self-splicing" may refer to the ability of an amino acid to self-splice. This self-splicing reaction may, therefore, result in a product comprising a protein of interest and a labeled amino acid and a product comprising two rejoined self-splicing amino acid sequences and a remaining portion of the enzyme. In specific embodiments "self-splicing amino acid sequences" refers to amino acid sequences of intein or fragments thereof. In embodiments a self-splicing amino acid sequence refers to one or more inteins or split inteins or parts or portions thereof, such as e.g., one or more N-intein and C-intein part(s) or portion(s).

"Inteins" are self-processing (or self-splicing) protein domains (or amino acid sequences), capable of catalyzing a process called 'protein splicing'. This involves the cleavage and formation of peptide bonds, enabling the ligation of adjacent exteins (external protein segments). The process of protein splicing does not require any external enzymes or cofactors, relying solely on the proper folding of the proteins involved. There are various types of inteins, including full-length, mini-inteins and split inteins, with the latter involving two separate fragments that require co-expression and subsequent association to restore functionality. Split inteins commonly comprise two parts or portions, namely an N-intein and C-intein part or portion, wherein the intein parts/portions are capable of a spontaneous and non-covalently assembly into the canonical intein structure to carry out protein splicing once they are brought into close proximity/close association. The versatility of inteins has made them valuable tools in protein engineering, with applications in basic research and potential therapeutic contexts with recent advancements in synthetic biology have led to the evolution of inteins with enhanced splicing efficacy, expanding their potential for diverse and complex functions, e.g., in chemical biology and protein engineering.

In the context of the present invention, the terminology 'autocatalytically self-splicing amino acid sequences' or 'self-splicing amino acid sequences' may refer preferably to inteins. Preferably the 'two' self-splicing amino acid sequences, or 'first' and 'second' self-splicing amino acid sequences, refer to the N- and C-intein (Int^{C}, Int^{N}) portions of a split inteins. In embodiments, the self-splicing of the Int^{C} and Int^{N} portions within the system according to the invention is preferably inhibited through a circular permutated enzyme (whose overall three-dimensional (3D) structure and functionality is preferably preserved), while said enzyme is capable of unfolding upon a labelling reaction (e.g., of its reactive amino acid (aa_{R})), such that the split intein parts are brought into close proximity upon said labelling reaction and their self-splicing is induced (triggered).

"Circular permutation" is a structural rearrangement of proteins in which the order of amino acids in the peptide sequence is altered while preserving the overall three-dimensional (3D) structure. This rearrangement results in different termini positions but maintains functional and structural integrity. Circular permutations can occur naturally due to evolutionary mechanisms or be artificially engineered for various applications. There are multiple mechanisms through which circular permutation can arise. One such mechanism occurring in nature is permutation by duplication, where a gene undergoes duplication, forming a tandem repeat. Start and stop codons are introduced, leading to the removal of redundant sequences. Another natural mechanism involves fission and fusion, where a single gene undergoes fission into two separate polypeptides that later fuse in an alternative arrangement, resulting in a circularly permuted protein. Additionally, in nature post-translational modifications can lead to circular permutation, where some proteins undergo spontaneous or enzymatic cleavage and rejoining without genetic changes. Particularly in the context of biochemistry and protein engineering artificial engineering also plays a role where laboratory techniques such as genetic recombination and site-directed mutagenesis facilitate the design of circularly permuted proteins, often used to modify stability, catalytic activity, and binding properties. In the context of the present invention a circular permutation of an enzyme (e.g., such as SNAP, e.g., circularly permutated between aa positions 132/133), which is located between two autocatalytically self-splicing amino acid sequences (e.g., split inteins) induces an inhibition, e.g., through (steric) hindrance, that prevents the self-splicing of said self-splicing amino acid sequences. In preferred embodiments the three-dimensional structure or folding of the circular permutated enzyme is (majorly) preserved, such that an unfolding-reaction of the enzyme may occur, which leads to a change in structure of the system according to the invention, thereby resolving the inhibition of the two split intein parts/portions by rearranging them in close proximity/association.

As used herein, the term "CLUSTER" refers to "Chemical Label-Unfold-Splice Technology Enables Recombination," a bio-orthogonal protein labeling system and associated methods developed to precisely label proteins with minimal perturbation to the biological system and protein functionality. The technology employs in embodiments a chimeric construct/system composed of a small label, e.g., a fluorescent peptide, in embodiments ~ 5 kDa in size, spliced onto a fusion protein of interest (POI). This method is particularly advantageous for labeling challenging targets, including transmembrane and disordered proteins, in living cells.

According to one embodiment of the invention, the CLUSTER construct is designed with a SNAP-tag (SNAP enzyme), which selectively interacts with benzylguanine (BG) substrates, forming a covalent bond at cysteine residue 145 (C₁₄₅ of SNAP). The SNAP-tag is preferably circularly permutated to incorporate a split intein (gp-41-1C) upstream of the reactive cysteine (aaR). Thereby, initially the circular permutated SNAP preferably induces an inhibition, e.g., via (steric) hindrance, thereby preventing the self-splicing of the split inteins. Upon the SNAP-tag's interaction with the BG substrate, the resulting conformational change of SNAP destabilizes the construct, thereby facilitating the meeting of the split inteins. This interaction triggers the splicing of the fluorescent peptide onto the protein of interest, effectively labeling the target protein. At the same time, the self-splicing intein mechanism enables the excision of the majority of the construct/system, such that size and mass of the final obtained labelled fusion protein of interest remains as small as possible and 'unnecessary' peptide portions of the construct/system (e.g., the majority of the SNAP enzyme and/or of the split inteins) are removed therefrom.

Herein 'SNAP' may refer to the (engineered) suicide enzyme *O*⁶-alkylguanine DNA alkyltransferase. SNAP can react with fluorophore-linked benzylguanine (BG) substrates forming a covalent bond on cysteine145 of SNAP (a reactive amino acid (aa_{R})), triggering unfolding of SNAP^{8,9}. Commonly used in biochemistry is the SNAP-tag, which refers to a self-labeling protein tag derived from a modified (engineered) form of human *O*⁶-alkylguanine-DNA alkyltransferase, which is still capable of covalently binding BG derivatives. SNAP or the SNAP tag (a 182-residue, 19.4 kDa polypeptide) can be fused to proteins of interest (POls) for targeted and specific labeling with synthetic ligands, such as fluorescent dyes. The reaction between SNAP(-tag) and BG derivatives is rapid, highly specific, and irreversible, forming a stable thioether bond between a reactive cysteine residue in the tag and the probe. Structural and functional modifications of SNAP, e.g., SNAP-tag, allow site-specific labeling in live and fixed cells without interfering with the biological activity of fusion proteins. Unlike fluorescent proteins, SNAP(-tag) exhibits superior photostability, quantum yield, and resistance to photobleaching, making it particularly suitable for single-molecule spectroscopy and super-resolution microscopy. Moreover, its labeling reaction is independent of the nature of the attached probe, enabling its use with a broad range of fluorescent, biotinylated, or affinity-based ligands. Several variations of SNAP(-tags) have been developed for specialized applications. CLIP-tag, e.g., an orthogonal variant, selectively binds O2-benzylcytosine (BC) derivatives instead of BG, allowing simultaneous labeling of two distinct proteins within the same cellular environment. In preferred embodiments herein 'SNAP' refers to engineered variants of the suicide enzyme SNAP, such as the SNAP tag. In general, a reactive amino acid aa_{R} of a SNAP-tag or a CLIP-tag may be Cys145. In general, a reactive amino acid aa_{R} of a Halo-tag may be Asp106. In general, a reactive amino acid aa_{R} of a SNAPtag2 may be Cys135. In embodiments such reactive amino acids enable / are capable of forming a covalent bond with a substrate, e.g., a substrate comprising a label, such as a fluorophore or similar. In embodiments, such reactions trigger the unfolding (of its 3D protein structure) of the enzyme.

"Covalently linked" may refer to in the context of the invention to the linkage of two molecules (e.g., an amino acid sequence and an enzyme and/or a label) through a covalent bond. A covalent bond is a chemical bond in which electrons are exchanged to form electron pairs between atoms, in chemistry it refers to the interatomic bond formed by the sharing of a pair of electrons between two atoms. This bond is formed by the electrostatic attraction of their nuclei to the same electrons. A covalent bond occurs when the bonded atoms have a lower total energy than that of widely separated atoms.

Herein, "nucleic acid" may preferably refer to DNA (deoxyribonucleic acid), gDNA (genomic deoxyribonucleic acid), RNA (ribonucleic acid), gRNA (genomic ribonucleic acid), mRNA (messenger ribonucleic acid) and cDNA (complementary deoxyribonucleic acid synthesized from RNA template), or any combination thereof. In a preferred embodiment the nucleic acid herein is DNA. Nucleic acid sequences refer herein to a consecutive array of nucleotides, wherein the nucleotides are distinguished by their nucleobases into guanine (G), adenine (A), cytosine (c) and thymine (T) in DNA and uracil (U) -instead of thymine- in RNA. A nucleic acid sequence may herein also refer to the sequence of consecutive letters (comprised of G, A, C and T or U) that represent the actual sequence of consecutive nucleic acids in a strand of DNA or RNA. This nucleic acid sequence may be biochemically and bioinformatically identified and characterized using DNA or RNA sequencing. The sequencing analysis may also involve the comparison of the obtained nucleic acid sequence and one or more reference nucleic acid sequences. As used herein, "nucleic acid" shall mean any nucleic acid molecule, including, without limitation, DNA, RNA and hybrids or modified variants thereof. An "exogenous nucleic acid" or "exogenous genetic element" relates to any nucleic acid introduced into the cell, which is not a component of the cells "original" or "natural" genome. Exogenous nucleic acids may be integrated or non-integrated or relate to stably transfected nucleic acids.

"Amino acids" (AA, aa) are chemical compounds with a nitrogen (N) containing amino group and a carbon (C) and oxygen (0) containing carboxylic acid group. The class of amino acids includes organic compounds that contain at least one amino group (-NH₂ or substituted -NR₂) and one carboxy group (-COOH) as functional groups. Selected α-amino acids are the natural building blocks of proteins, which are linked together to form chains by the carboxy group of one amino acid forming a peptide bond with the amino group of the next. The amino acids thus linked to form a polymer differ in their side chains and together determine the shape with which the polypeptide then unfolds in the aqueous environment to form the native protein. This biosynthesis of proteins takes place in all cells at the ribosomes according to genetic information, which is available in the form of mRNA. The base sequence of the mRNA encodes the amino acid sequence in triplets, with one base triplet each representing a codon that stands for a specific proteinogenic amino acid.

A "peptide" is an organic compound containing peptide bonds between amino acids. Accordingly, a "peptide" or "peptide sequence" refers to the sequence (and/or identity) of consecutively linked amino acids of a peptide. According to their number, oligopeptides with few are distinguished from polypeptides with many amino acids. Long polypeptide chains are also called "proteins", especially those formed by protein biosynthesis. As used herein, "polypeptide" shall mean both peptides and proteins. In this invention, the polypeptides may be naturally occurring or recombinant (i.e., produced via recombinant DNA technology), and may contain mutations (e.g., point, insertion and deletion mutations) as well as other covalent modifications (e.g., glycosylation and labelling (via biotin, streptavidin, fluorescein, and radioisotopes), non-proteinogenic amino acids, artificial amino acids and/or other molecular bonds to additional components.

Sequence variants of nucleic acids, proteins and/or other biomolecules of the claimed system for or example defined by the claimed % sequence identity, that maintain the said properties of the invention, are also included in the scope of the invention. Such variants showing alternative sequences but maintain essentially the same properties and functionality as the specific sequences provided are known as functional analogues, or as functionally analogous. In general, sequence identity relates to the percentage of identical amino acids or nucleotides when carrying out a sequence comparison or alignment.

Protein sequence modifications, which may occur through substitutions, are also included within the scope of the invention. Substitutions as defined herein are modifications made to the amino acid (aa) sequence of the protein, whereby one or more amino acids are replaced with the same number of (different) amino acids, producing a protein which contains a different amino acid sequence than the primary protein, preferably without significantly altering the function of the protein.

In general, the non-polar amino acids Gly, Ala, Val, Ile and Leu; the non-polar aromatic amino acids Phe, Trp and Tyr; the neutral polar amino acids Ser, Thr, Cys, Gln, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups. Substitution variants have at least one amino acid residue in the antibody molecule removed and a different residue inserted in its place.

| **Original residue** | **Preferred conservative substitutions** | **Examples of exemplary substitutions** |
|---|---|---|
| Ala (A) | Val | Val; Leu; Ile |
| Asg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn, Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

Variants of proteins or nucleic acids: "Variants" of proteins, peptides or nucleic acids, as defined in the context of the present invention, may be generated that have a or nucleic acids or amino acid sequence differing from the original sequence in one or more mutation(s), such as one or more substituted, inserted and/or deleted or nucleic acid(s) or amino acid(s). In embodiments, the sequence variation described here with respect to substitutions and/or percentage identity, may apply to any one or more embodiments, described throughout the application as a whole.

Preferably, any variants or fragments have the same biological function or specific activity compared to the native full-length nucleic acid or protein.

Fragments of proteins: "Fragments" of proteins or peptides in the context of the present invention may typically comprise a sequence of a protein or peptide as defined herein, that is, with respect to its amino acid sequence (or its encoded nucleic acid) molecule), N-terminally and/or C-terminally truncated compared to the amino acid sequence of the original (native) protein (or its encoded nucleic acid molecule).

In this context, a fragment of a protein may typically comprise an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98% or 99%, preferably of at least 70%, more preferably of at least 80%, even more preferably of at least 85%, even more preferably of at least 90% and most preferably of at least 95% or even 97%, having an amino acid sequence of the respective naturally occurring full-length protein.

It is understood that substituents and substitution patterns of the compounds described herein can be selected by one of ordinary skill in the art to provide compounds that are chemically stable and that can be readily synthesized by techniques known in the art and further by the methods set forth in this disclosure.

Variation in length of the amino acid sequences and encoding nucleic acids as described herein is also encompassed by the present invention. A skilled person is capable of providing natural or artificial amino acid sequence variants that are longer or shorter than the specific sequences of SEQ ID NO 1 to 32, which will still exhibit sufficient similarity to the natural proteins in order to provide the desired functional capabilities described herein. For example, shorter variants of the amino acid sequences of the respective protein, peptide or enzyme of the present system, e.g., of SEQ ID NO 1 to 32 comprising 1, 2, 3, 4, 5, 10, 15, 20, 25, 30, 35, 40, 45 or 50 amino acids less than the full-length form may also exhibit effective functionality, as described herein. For example, longer variants of the of the present system, e.g., of SEQ ID NO 1 to 32, comprising 1, 2, 3, 4, 5, 10, 15, 20, 25, 30, 35, 40, 45 or 50 amino acids of a respective protein, peptide or enzyme of the present system more than the natural length form may also exhibit effective functionality, as described herein.

In some embodiments of the invention the peptide, preferably according to sequences disclosed herein, may comprise a 0 to 10 amino acid addition or deletion at the N and/or C terminus of a sequence.

As used herein the term "a 0 to 10 amino acid addition or deletion at the N and/or C terminus of a sequence" means that the polypeptide may have a) 0, 1, 2, 3, 4, 5, 6, 7, 8, 9 or 10 additional amino acids at its N terminus and 0, 1, 2, 3, 4, 5, 6, 7, 8, 9 or 10 amino acids deleted at its C terminus or b) 0, 1, 2, 3, 4, 5, 6, 7, 8, 9 or 10 additional amino acids at its C terminus and 0, 1, 2, 3, 4, 5, 6, 7, 8, 9 or 10 nucleotides deleted at its N terminus, c) 0, 1, 2, 3, 4, 5, 6, 7, 8, 9 or 10 additional amino acids at its N terminus and 0, 1, 2, 3, 4, 5, 6, 7, 8, 9 or 10 additional amino acids at its N terminus or d) 0, 1, 2, 3, 4, 5, 6, 7, 8, 9 or 10 amino acids deleted at its N terminus and 0, 1, 2, 3, 4, 5, 6, 7, 8, 9 or 10 amino acids deleted at its C terminus.

As used herein, a "label" is any compound, substance, molecule, atom, chemical modification or any other entity or structure, that makes possible the identification or determination of the protein of interest, for example and without limitation, in any analytical and/or biological system. In embodiments, the label may be considered a "marker", "dye" or "tag" that facilitates detection and/or purification of the POI. In embodiments, a "label" preferably comprises a fluorescent or luminescent label, a biotin label, a chromogenic label, an MRI label, a metal label, dyes, radionuclides, or iodine-125 (125I). Fluorescence is a form of luminescence that occurs when matter emits light of a certain wavelength after absorbing electromagnetic radiation.

A fluorophore (or fluorochrome, which is similar to a chromophore) is defined as a fluorescent chemical compound that is capable of reemitting light when excited by light. Fluorophores for use as fluorescent or luminescent labels include, without being limited thereto, rhodamine and derivatives, lissamine DAPI, Hoechst 33258, fluorescein, 5-bromomethylfluorescein and derivatives, R-phycocyanin, B-phycoerythrin, R-phycoerythrin, Lucifer Yellow, 7-Me2N-coumarin - 4-acetate, IAEDANS, 7-OH-4-CH3-coumarin-3-acetate, monobromobiman, Pyrene trisulfonates such as Cascade Blue and monobromotrimethyl ammoniobiman, Texas Red, Rhodamine Green, Oregon Green 30 488, Oregon Green 514, 7-NH2-4CH3-25-coumarin-3-acetate (AMCA), FAM, TET, CAL Fluor Gold 540, JOE, VIC, Quasar 570, CAL Fluor Orange 560, Cy3, NED, Oyster 556, TMR, CAL Fluor Red 590, HEX, ROX, CAL Fluor Red 610, LC Red 610, CAL Fluor Red 610, LC Red 640, CAL Fluor Red 635, LC Red 670, Quasar 670, Oyster 645, LC Red 705, BODIPY FL, Cal Gold, BODIPY R6Gj, Yakima Yellow, Cal Orange, BODIPY TMR-X, JOE, HEX, Quasar-570 /Cy3, TAMRA, Rhodamine Red-X, Redmond Red, BODIPY 581/591, Cy3.5, Cal Red/Texas Red, BODIPY 630/665-X, BODIPY TR-X , Quasar-670/Cy5, Pulsar-650, Cy5, Cy5.5, Dy590, Dy490, Dy636, Dy682, Atto-488, Atto-532, Atto-Rho-6G, Atto-Rho101, Atto-647N, Atto-680, BMN-488, BMN-505, BMN-536, BMN-562. Indirect labels, such as chromogenic labels, include various enzymes well-known in the art, such as horseradish peroxidase (HRP), alkaline phosphatase (AP), β-galactosidase, urease, and the like.

In fluorescence microscopy, a specific wavelength or wavelengths of light are employed to illuminate the specimen, which can be absorbed by the fluorophores, resulting in the emission of light of longer wavelengths. Ideally, a spectral emission filter is utilized within the microscope to separate the excitation light from the fluorescence emitted by the specimen. Fluorescence imaging encompasses a range of techniques for visualizing fluorescent proteins or fluorescence dyes as molecular markers of biological structures and functions. Fluorescence imaging enables the detection, monitoring and investigation of a variety of processes and functions at the molecular level, including the localization or presence of gene/protein expression, protein-protein interactions and molecular signaling pathways within the cellular and tissue context. Fluorescence imaging techniques include microscopy, Fluorescence Activated Cell Sorting (FACS), imaging probes and spectroscopy.

### FIGURES

The invention is further described by the following figures. These are not intended to limit the scope of the invention but represent preferred embodiments of aspects of the invention provided for greater illustration of the invention described herein.

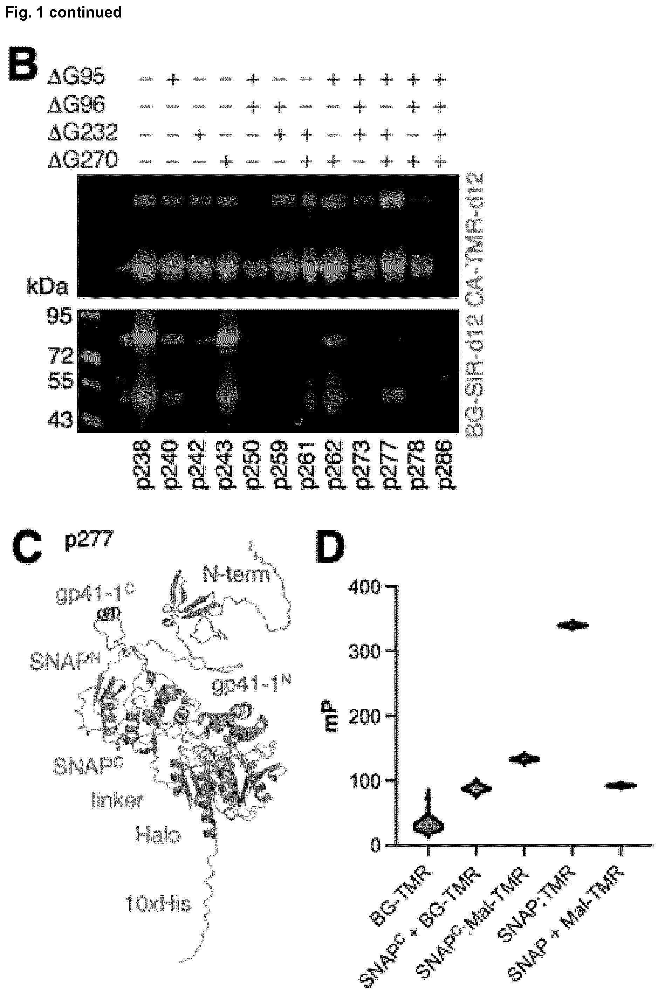

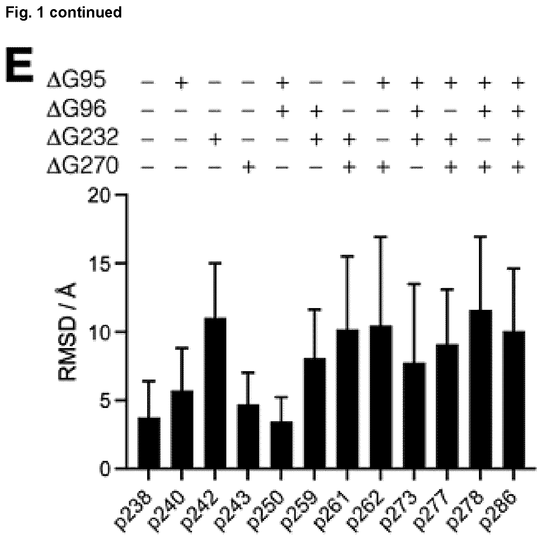

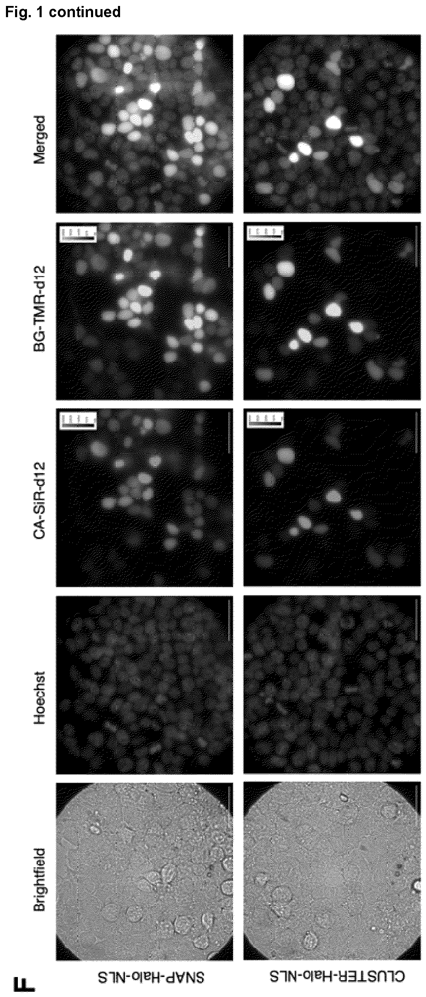

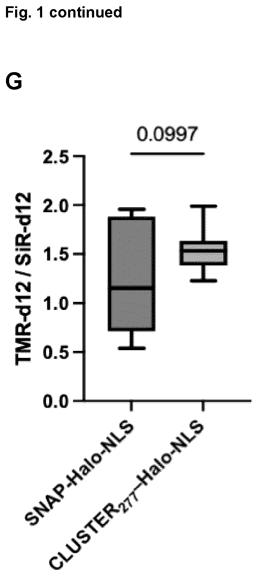

**Figure 1****: CLUSTER design and development. A)** SNAP-tag is endowed with an N-terminal Int^{N} (gp41-1N) and circularly permutated with an Int^{C} (gp41-1C) between position 132-133, upstream of the reactive Cys145 (aaR) that resides on the C-terminal SNAP locus. A Halo-tag is fused for expression control and normalization with a CA-TMR-d12 fluorophore. Upon SNAP-tag reaction with its BG substrate, the construct becomes less stable and the inteins meet for a splicing reaction, transferring the labeled C-terminal SNAP sequence on a protein of interest. i) Split inteins consist of an N- and C-terminal part, which splice the protein sequences they are attached to give a new fusion and a full intein. ii) The SNAP-tag can be covalently and specifically labeled on Cys145 (aa_{R}) with a bio-orthogonal BG-substrate, transferring a cargo of choice. iii) "CLUSTER": SNAP is equipped with split inteins (Int^{N} and circularly permutated Int^{C}), fused to a protein of interest. hAGT will unfold once it is labeled covalently with a BG-substrate. In turn, Int^{C} will be able to fold and subsequently react with the upstream Int^{N} to splice the fluorophore containing, small amino acid sequence (~4 kDa) onto a POI. In other words, a large protein part is the leaving group, and a minimal tag of includes the fluorophore cargo is transferred. iv) Supporting the rationale from (iii), AlphaFolded SNAP-IntC (black-grey) reveals that crucial parts of IntC are disordered (compared to beta-strands in IntC in orange), suppressing splicing before hAGT reacted with a substrate. **B)** SDS-PAGE gel under a fluorescent scanner after CLUSTER-transformed and labeled E. coli were allowed to express different constructs over night before harvesting. Construct p277 (CLUSTER₂₇₇) shows clean labelling of only spliced product. **C)** Alpha-fold of p277 revealing the separation of the split inteins. **D)** Fluorescent polarization assay to mechanistically determine labeled protein dynamics show SNAPC:Mal-TMR with more degrees of freedom compared to SNAP:TMR. **E)** RMSD values of all CLUSTER constructs after alpha-folding and alignment. **F)** Live HEK293 cells transiently transfected with SNAP-Halo-NLS or CLUSTER-Halo-NLS and stained with CA-SIR-d12 and BG-TMR-d12. Quantification is depicted in **G).**

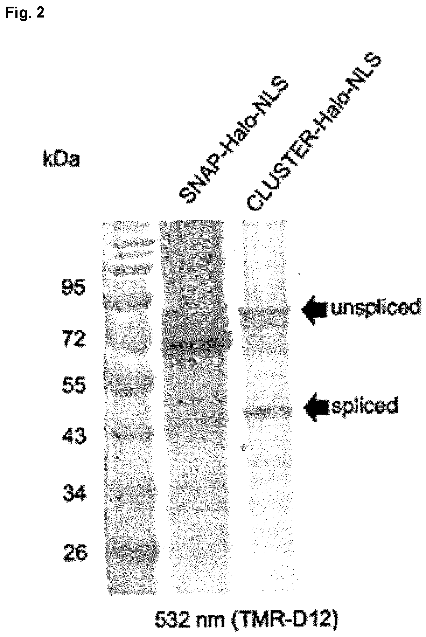

**Figure 2****: SDS-PAGE of cells form** **Fig. 1F****,** fluorescent scan shows spliced CLUSTER fragments.

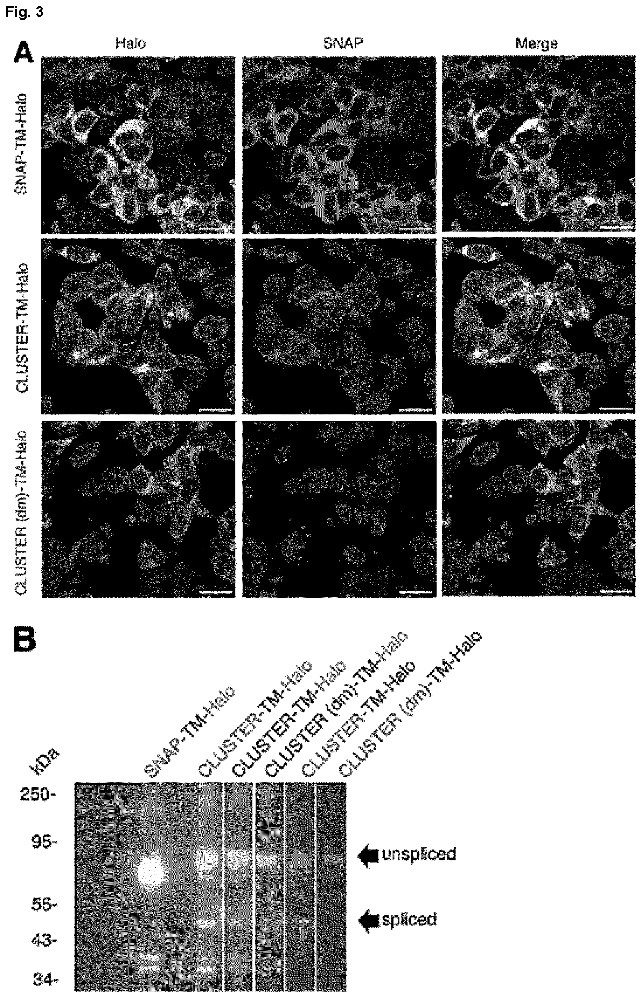

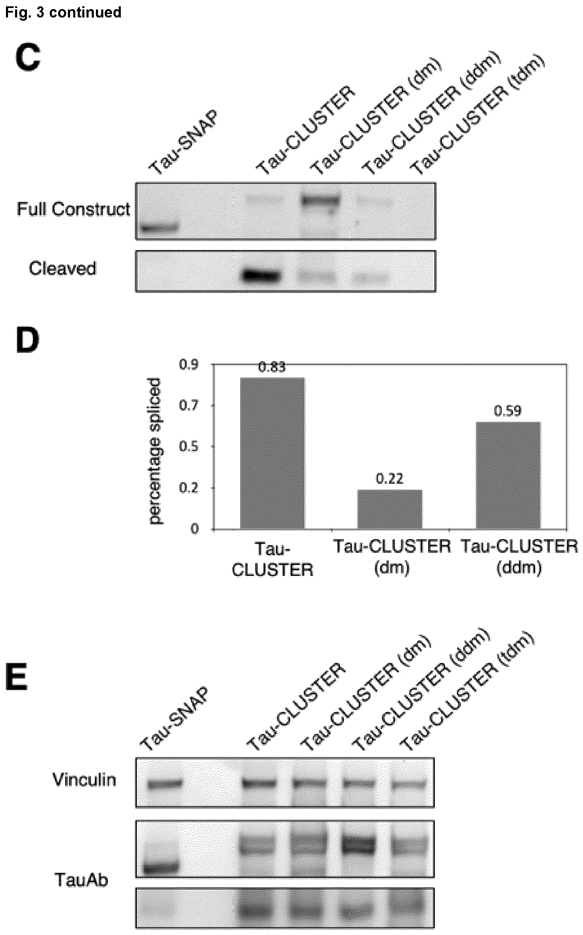

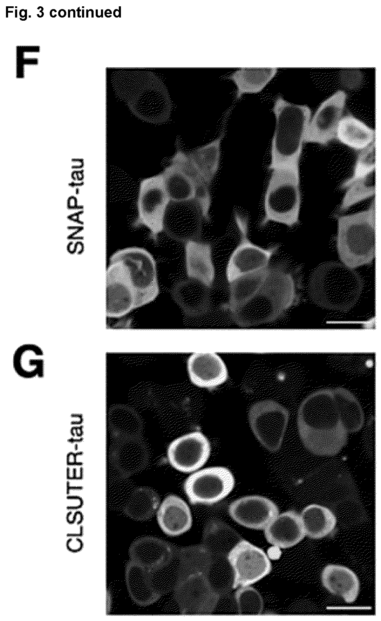

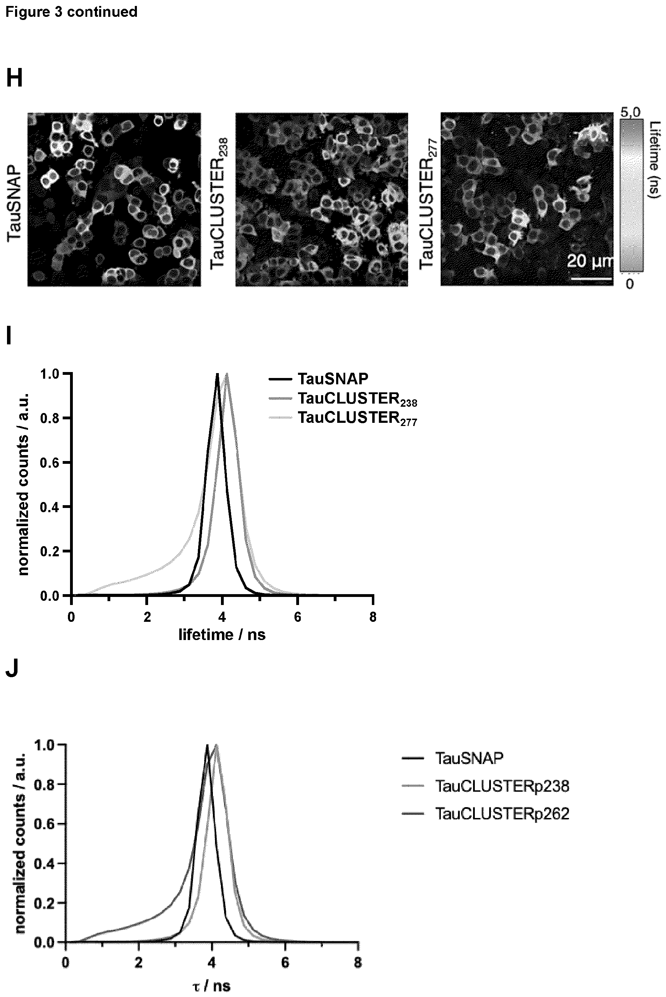

**Figure 3****: The present system and method enable labelling of transmembrane and disordered proteins in live cells. A)** HEK293 cells transiently transfected with SNAP-TM-Halo, CLUSTER₂₃₈-TM-Halo, or CLUSTER_{dm} (deadmutant)-TM-Halo labeled with CA-TMR_d12 and BG-SiR-d12. CLUSTER_{dm} is a non-splicing dead mutant of CLUSTER₂₇₇, facilitated by mutation of the nucleophilic split intein residue to alanine. **B)** SDS-PAGE of cells form (**A**), fluorescent scan shows spliced CLUSTER fragments. **C)** Fluorescent scan of SDS-PAGE from Tau-fusions. Vinculin served as loading control. From left to right lane: TauSNAP, TausCLUSTER₂₃₈, TauCLUSTER_{dm}, TauCLUSTER₂₆₂, TauCLUSTER₂₇₇, **D)** Quantification of spliced fragments from (C). **E)** Western blot for Tau of Tau-fusions transfected in HEK293T cells. From left to right lane: TauSNAP, TausCLUSTER₂₃₈, TauCLUSTER_{dm}, TauCLUSTER₂₆₂, TauCLUSTER₂₇₇. **F**) Fluorescent lifetime imaging of HEK293T cells transfected with SNAP-tau and labeled with BG-SiR-d12. **G)** As for (**F**) but cells were transfected with CLUSTER-tau. **H)** Fluorescent lifetime images of HEK293T cells transfected with Tau-fusions. **I)** Fluorescent life time histograms of (**H**). **J**) Fluorescent lifetime histograms of HEK293T cells transfected with Tau-fusions .

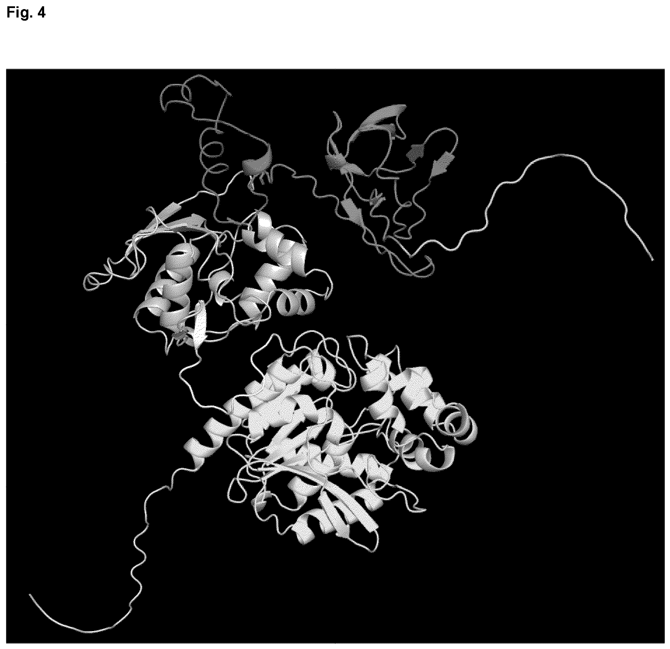

**Figure 4****: The figure depicts alphafolded p238 with grayscale coding.**

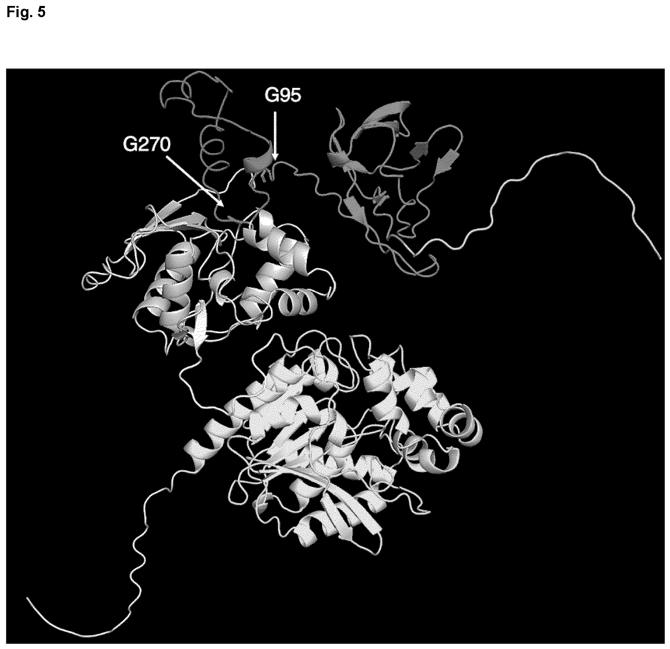

**Figure 5****: Several mutations were tested on the bacterial expression system.** In one experiment a construct did perform best comprising the mutant p262, wherein the introduction of mutations into the mutant p238 yielded, among others, p262, a preferred embodiment of the present invention. The figure shows deletions of linker glycines G95 and G270.

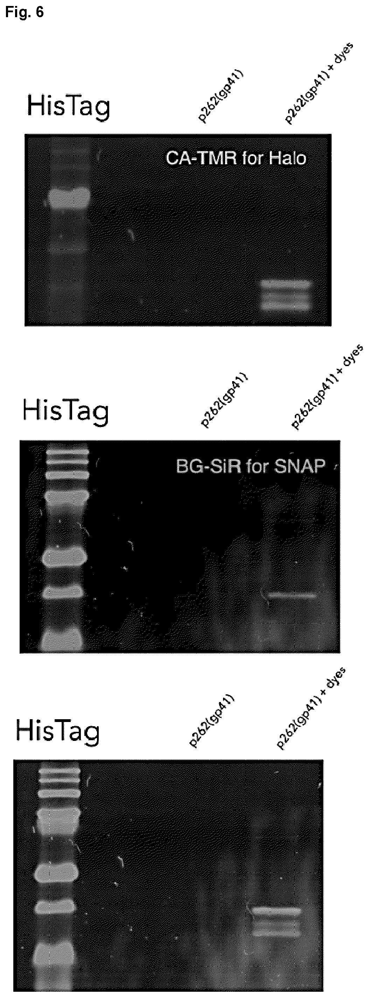

**Figure 6****: Expression of p262 in the presence of CA-TMR-d12 (for Halo-tag labelling) and BG-SiR-d12 (for SNAP-tag labelling) over night after IPTG induction.** P262 constitutes: gp41-1N-SNAPf[132GG-133]gp41-1C_ΔG95ΔG270. NiNTA purification and SDS page reveals labeled and spliced CLUSTER product under fluorescence gel scanner.SDS-PAGE of NiNTA purified p262.

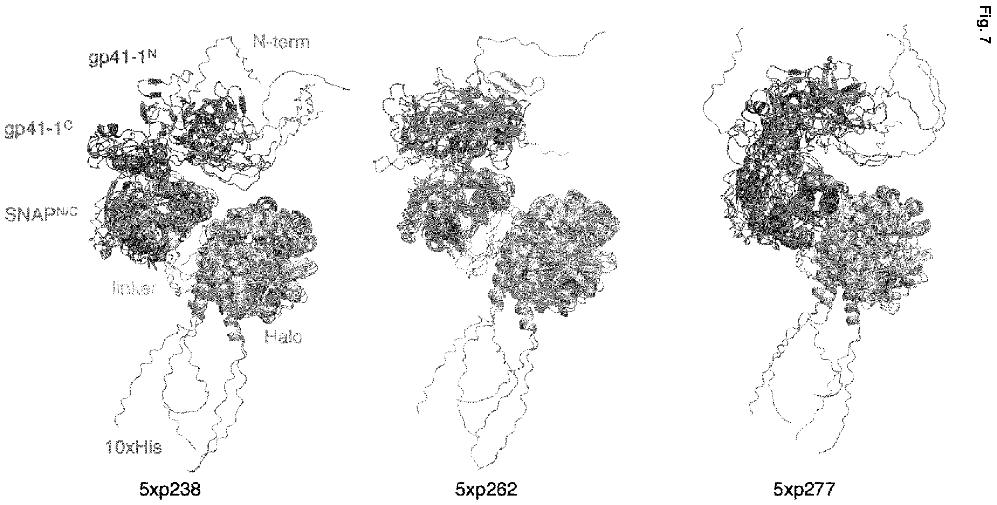

**Figure 7****: Comparison between p238, p262 and p277.** P277 constitutes: gp41-1N-SNAPf[132GG-133]gp41-1C_ΔG95ΔG232ΔG270. AlphaFolding and alignment of 5 folded structures shows differences between p238, p262 and p277. Note the partially interacting split intein parts in p262 and p277, indicating better interaction upon SNAP labelling.

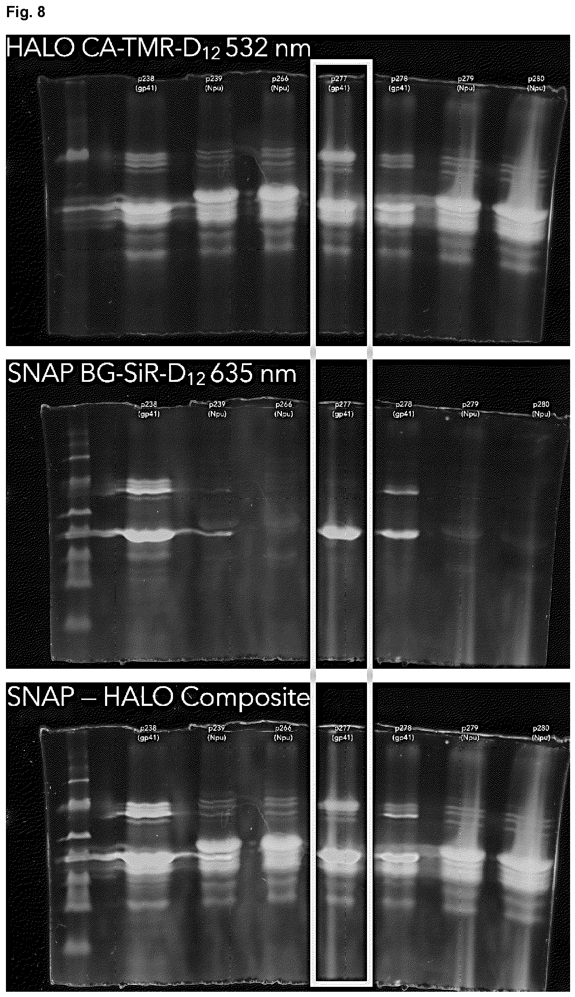

**Figure 8****: Comparison between p238, p262 and p277 among other mutants in an SDS-PAGE without NiNTA purification.** Order of mutants in SDS-PAGE (left to right): p238 (gp41), p239 (Npu), p266 (Npu), p277 (gp41), p278 (gp41), p279 (Npu), p280 (Npu). Of note is the clean labelling of only spliced product for p277 (white box).

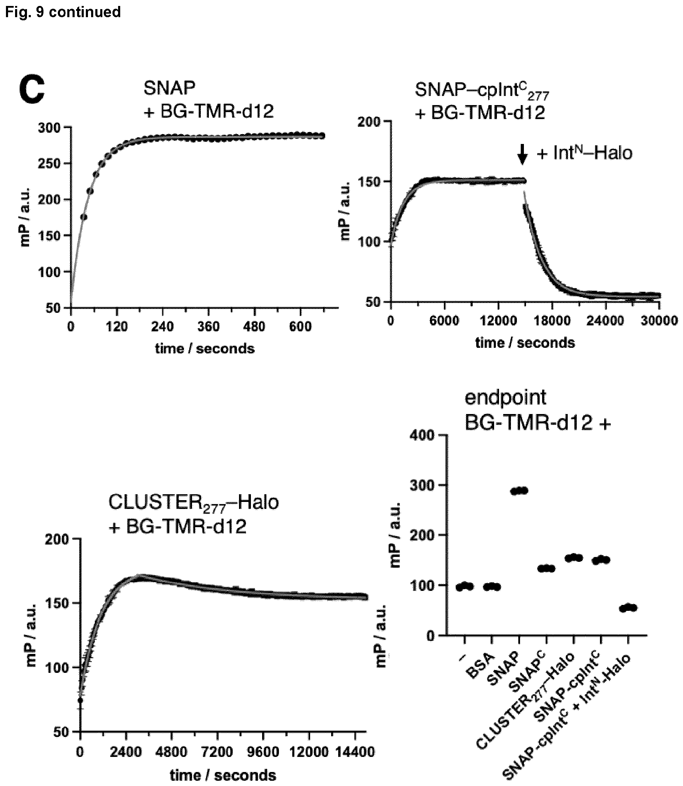

**Figure 9****: CLUSTER design and development. A)** SNAP-tag is endowed with an N-terminal Int^{N} (gp41-1^{N}) and circularly permutated with an Int^{C} (gp41-1^{C}) between position 132-133, upstream of the reactive cysteine145. Upon SNAP-tag reaction with its BG substrate, the construct becomes less stable and the inteins meet for splicing, transferring the labelled C-terminal SNAP sequence on a protein of interest. **B**) SDS-PAGE gel under a fluorescent scanner after CLUSTER-Halo-transformed and labelled *E. coli.* Construct p277 shows clean labelling of only spliced product. **C**) Fluorescent polarization assay to mechanistically determine protein labelling and dynamics.

### EXAMPLES

The invention is further described by the following examples. These are not intended to limit the scope of the invention but represent preferred embodiments of aspects of the invention provided for greater illustration of the invention described herein.

### Example 1

In the present example, the inventors tested an exemplary embodiment of the system and method evidencing the surprising capability of the present system and methods to i) minimize tag-size, in a ii) controllable manner regarding space and experiment time, its iii) bio-orthogonal manner, and its iv) applicability in living cells and with regard to iv) using synthetic fluorophores. This embodiment of the present approach utilizes the engineered suicide enzyme *O*⁶-alkylguanine DNA alkyltransferase termed 'SNAP'. SNAP can react with fluorophore-linked benzylguanine (BG) substrates forming a covalent bond on cysteine145.^{8,9}

The inventors initially analyzed two different intein incorporations, the gp41-1 and the AvaN-NpuC inteins, in both bacterial (part 1) and mammalian (part 2) expression systems. In said experiments the inteins were introduced to a construct of SNAP-Halo, as this control construct allowed easy evaluation of the system, where the fluorescence of Halo stays stable but the changes on the systems could be monitored. Figures 4 and 5 depict two exemplary constructs according to non-limiting exemplary embodiments of the present system. The analyzed constructs comprised two different inteins as respective autocatalytic self-splicing portions (sequences) and were expressed recombinantly in bacterial cells. Several mutations were tested on the bacterial expression system, and the experiments revealed as promising constructs the mutant p262 (Fig. 5) originating from construct p238 (Fig. 4) by further deleting G95 and G270. The most promising constructs generated were P262 and P277. P262 comprised gp41-1N-SNAPf[132GG-133]gp41-1C_ΔG95 Δ G270 and P277 comprised gp41-1N-SNAPf[132GG-133]gp41-1C_ Δ G95 Δ G232 Δ G270.

The expression of p262 in the presence of CA-TMR-d12 (for Halo-tag labelling) and BG-SiR-d12 (for SNAP-tag labelling) was analyzed over night after IPTG induction. NiNTA purification and SDS page revealed successful labeled and spliced system-products under fluorescence gel scanner (Fig. 6).

Figure 7 illustrates AlphaFolding and alignment of 5 folded structures showing differences between the constructs p238, p262 and p277, wherein the partially interacting split intein portions in p262 and p277, indicate a likely further improved interaction of the split intein portions upon SNAP labelling.

### Application of an embodiment of the present invention (CLUSTER) in eukaryotic, live HEK cells

For heterologous expression in mammalian systems the inteins were introduced to the N-terminal SNAP tag containing a transmembrane domain (TM) followed by a C-terminal Halo-tag (referred to as the SNAP-TM-Halo construct). The SNAP-TM-Halo construct with an N-terminal signal sequence and the construct, where SNAP is substituted with the p262 system variant (also referred to as the "CLUSTER-TM-Halo construct"), were expressed in HEK293T cells. The signal peptide in these constructs is supposed to traffic the expressed protein to the outer cell membrane upon transfection with lipofectamine. Observed by confocal imaging we detect localization of SNAP and Halo substrates (i.e. BG-SiR-d12 and CA-TMR-d12 respectively) on the cell's periphery (cell membrane localized proteins) and the ER (newly translated protein) (Fig. 3A). After imaging the cells were processed for protein extraction. As observed on SDS PAGE gel of the cell lysates, spliced products of the CLUSTER constructs are observed and detected with both SNAP and Halo substrates (Fig. 3B). Most convincingly, the dead mutant of the CLUSTER construct which deteriorates splicing shows no intein mediated splice product as shown by imaging and on SDS PAGE (Fig. 8A, B).

In the following subsequent experiments, gp41-1 were used as autocatalytic self-splicing portions (sequences).

The inventors circularly permutated SNAP between positions 132/133 to include a trans-splicing split intein^{C} (gp-41-1^{C 25}) peptide upstream of the reactive Cys145, thereby preferably creating a steric hindrance of the self-splicing of the inteins. Upon SNAP-labelling, gp-41-1^{C} will become accessible to its natural interaction partner, gp-41-1^{N}, that resides between the N-terminus of SNAP and the POI. When the stoichiometric split parts meet, they splice the fluorophore containing peptide fragment (48 amino acids, i.e. 5.2 kDa excluding cargo) onto the POI. This engineered construct (system) is termed herein "CLUSTER", standing for Chemical Label-Unfold-Splice Technology Enables Recombination (Fig.1A).

The inventors created a construct (gp-41-1^{N}-SNAP^{N}-gp-41-1^{C}-SNAP^{C}-Halo-10xHis, CLUSTERₚ₂₃₈-Halo-10xHis) (Fig. 1A, Fig.S1A) as a starting point and subcloned it in a bacterial vector for screening in an *E. coli* ²⁶ for labelling of SNAP (BG-SiR-d12) and Halo (CA-TMR-d12), the latter for expression control and normalization. After outgrowing overnight, cells were harvested and lysed and subjected to SDS-PAGE to observe both spliced and non-spliced, tagged proteins under a fluorescent scanner.

Encouraged by this, the inventors performed screen to delete the glycine linker (i.e G95, G96, G232, G270 and combinations thereof) to find mutant CLUSTERₚ₂₇₇, which was only SiR-labeled at the lower molecular weight band, indicating splicing upon labelling (Fig.1B). Alpha-folding of CLUSTERₚ₂₇₇-Halo reveals the separation between the split inteins upon folded SNAP-tag (Fig. 1C). When testing SPPS-synthesized SNAP^{C}:Mal-TMR-d12-fragment against full length SNAP:TMR-d12, fluorescence polarization was ~5-fold lower after background subtraction, indicating less structured features (Fig.1D). Importantly, BG-TMR-d12 was unable to react with SNAP^{C}, confirmed by mass spectrometry. Calculating RMSD values of five, each alpha-folded CLUSTER constructs depicts p277 with a higher value, however, does not fully explain its binary reaction mechanism (Fig. 1E).

In addition, the inventors performed fluorescence polarization on NiNTA purified CLUSTERₚ₂₇₇-Halo-10xHis and, when compared to CLUSTER_{p277dm}-Halo-10xHis, observed beneficial splicing kinetics (Fig.1F, 9C)). To provide a broader scope, the inventors performed successful CLUSTER labelling with different fluorophores from the green to far-red regime.

**Table 2: Kinetic models used for FP data fitting.**

| | **reaction** | **model** |
|---|---|---|
| labelling | Prot + Dye → Prot: Dye | FP(*t*) = *a* (1 - *e^{-kt}*) + *b* |
| splicing | Prot: Dye → Term: Dye + Bulk | FP(*t*) = *a e^{-kt}* + *b* |

The recorded data, as well as the anticipated reaction pathway, are presented in Figures 1 and 9, whereas obtained kinetic constants, as well as *goodness-of-fit* parameters, are shown in Table 3.

**Table 3: Determined reaction constants of specific model reactions.**

| **system** | **reaction** | **k / s⁻¹** | **RMSD / mP** | **R** |
|---|---|---|---|---|
| SNAP:BG-TMR-D₁₂ | labelling | 22.24 x 10⁻³ | 1.387 | 0.998 |
| C-277:BG-TMR-D₁₂ | labelling | 0.92 x 10⁻³ | 1.398 | 0.998 |
| | splicing | 0.33 x 10⁻³ | 0.477 | 0.989 |
| C-340:BG-TMR-D₁₂ | labelling | 0.72 x 10⁻³ | 1.347 | 0.992 |
| | splicing | 0.53 x 10⁻³ | 1.314 | 0.997 |

Next, the inventors applied the encouraging *in vitro* observations in live cell imaging. As proof of concept, they subcloned CLUSTER from p238 onto a Halo-tag bearing nuclear localization sequence (NLS) and transfected HEK293 cells (Fig. 1F). SNAP-Halo-NLS served as a control, and indeed for both cases a good signal overlap from the nucleus was observed. Harvesting these cells and running an SDS-PAGE revealed spliced fragments only in the CLUSTER-expressing cells (Fig. 2).

Next, a transmembrane domain (TM) was used, followed by a C-terminal Halo-tag (referred to as the CLUSTER-TM-Halo construct). A SNAP-TM-Halo construct was also used as a control. The signal peptide in these constructs traffics the expressed protein to the outer cell membrane upon transfection with lipofectamine. Constructs were expressed in mammalian HEK293T cells. Using confocal imaging localization of SNAP and Halo substrates was detected (i.e. BG-SiR-d12 and CA-TMR-d12 respectively) on the cell's periphery (cell membrane localized proteins) and the ER (newly translated protein) (Fig. 3A).

After imaging, the cells were processed for protein extraction. As observed on SDS PAGE gel of the cell lysates, spliced products of the 'CLUSTER' constructs are observed and detected with both SNAP and Halo substrates (Fig. 3A). Most convincingly, the dead mutant of the 'CLUSTER' construct which deteriorates splicing shows no intein mediated splice product as observed by imaging after transfection and on SDS PAGE of cell lysates.

To further demonstrate feasibility, the inventors performed Western blotting on cell lysates and observed splicing (Fig. 3C). Quantification of spliced to non-spliced signal indicates that 83% of expressed protein conjugates with BG-SiR-d12 in mammalian cells. To further test whether CLUSTER also functions on the more challenging disordered part of the proteome, we subcloned CLUSTER from p277 onto the tau protein. This protein is intrinsically disordered, adopting an ensemble of different conformations which give rise to multiple functions and involvement in many severe neural pathologies. Transfection of CLUSTER₂₇₇-tau and SNAP-tau in HEK293T cells and subsequent labelling revealed comparable expression levels by confocal microscopy.

Moreover, SDS-page was used to detect the extent of splicing of the CLUSTER₂₇₇-tau by measuring BG-SiR-d12 fluorescence of cell lysates expressing the SNAP-tau and CLUSTER₂₇₇-tau constructs. Two bands, at 95kDa and 40kDa presenting the unspliced and spliced CLUSTER₂₇₇-tau constructs are detected. Further to verify the presence of tau western blot analysis similarly detected the unspliced and spliced CLUSTER₂₇₇-tau fragments (Fig. 3D).

From the optimization screen conducted *in vitro,* the most effective double and triple mutants were also cloned onto tau. Interestingly, while all "CLUSTER" constructs are expressed in cells, CLUSTER₂₇₇ and the glycine double mutant show most efficient labelling of spliced fragments. We further used the glycine double mutant to conduct optimization of expression and labelling. Here cells transfected with CLUSTER₂₇₇-tau (double mutant) for 24 h were compared for the addition of SNAP substrate, BG-SiR-d12, either 1 h or 24 h after transfection.

For the 1 h condition, cells were stained overnight, while for the 24 h cells were stained for 1 h. This showed that the time required for staining CLUSTER₂₇₇-tau (double mutant) in cells is minimum.

To determine the spliced and unspliced populations of CLUSTER₂₇₇-tau in live cells and to determine whether they conformationally differ, fluorescence lifetime imaging (FLIM) was used. FLIM reports on kinetic parameters, independent of probe concentration and intensity informing about protein structure and microenvironment localization.

As expected, the newly translated protein was detected in the ER expressing unspliced CLUSTER₂₇₇-tau with a lifetime *τ* _{BG-SiR-d12}, = 4 ns, which is significantly higher to the lifetime of SNAP-tau (*τ* _{BG-SiR-d12}, = 3 ns) expressing cell. In the same cells expressing CLUSTER₂₇₇-tau, in the cytosol the BG-SiR-d12 has a longer lifetime likely representing the sliced CLUSTER₂₇₇-tau. This was further confirmed with timelapse FLIM imaging monitoring.

Overall, the inventors could show that the present method (CLUSTER) can be efficiently used for labelling challenging protein systems in living cells, such as transmembrane and dynamic intrinsically disordered proteins. Elegantly, the CLUSTER cassette can be inserted in embodiments in frame in between any DNA sequence to include a small, fluorescent label at any desired protein position (between or within functional domains) enabling a strictly stoichiometric reaction. Moreover, the exogenous addition of the BG-fluorophore provides exceptional temporal control for labelling.

The inventors envisage the present method and system for enabling genetic fusion to POIs that undergoes a specific and rapid reaction with synthetic fluorophore-coupled substrates. With its small size after splicing the fluorophore-containing sequence with a size of 5.2 kDa + cargo, CLUSTER is significantly smaller than popular proteins like the HaloTag (33 kDa), SNAP-tag (20 kDa) and FPs (27 kDa).⁷ Still bigger than unnatural amino acids,¹¹ however, the present method and system overcomes other challenges when applied to live cells in terms of costs, toxicity, and putative off-target incorporation ¹². In contrast, other methods, e.g. Lap-tag/LpIA^{W37V} ref¹³ or TC-tag/ReAsH¹⁴, are restricted on the cargo. Labelling based on the present method and system is also superior for all single molecule imaging approaches where the concentration of fluorescence is critical. The substrate conjugation and slicing reactions allow for controlled labelling and protein tracing.

### Example 2

Fluorescence imaging of proteins of interests (POls) requires specific labeling with chromophores, often achieved as fusion proteins.¹⁻³ Fluorescent proteins (FPs) are inferior to organic fluorophores that display augmented brightness and photostability,^{4,5} and which can be specifically and rapidly reacted with self-labeling protein tags (SLPs)⁶, like the HaloTag (33 kDa) and SNAP-tag (20 kDa). Comparably large in size to FPs (27 kDa),⁷ folded tags, however, may disrupt conformations, functions and dynamics of POls.

Herein, is described a conceptually new method that i) minimizes tag-size, is ii) controllable in space and time, is iii) bio-orthogonal, as such iv) applicable in live cells and v) uses synthetic fluorophores. The approach utilizes the SNAP-tag, which reacts with benzylguanine (BG) substrates, forming a covalent bond on cysteine145.^{8,9} The inventors circularly permutated SNAP between positions 132/133 to include a split intein^{C} (gp-41-1^{C}) upstream of the reactive Cys145. Upon SNAP-labelling, gp-41-1^{C} will become accessible to its natural interaction partner, gp-41-1^{N}, that resides on the N-terminus of SNAP. When the stoichiometric split parts meet, they splice the fluorophore containing peptide (48 amino acids, i.e. 5.2 kDa excluding cargo) onto the POI. Such engineered constructs are termed in the following 'CLUSTER' (see also above).

The inventors created a construct (CLUSTERₚ₂₃₈-Halo-10xHis) as a model system for screening in *E. coli.* BG-SiR-d12 and CA-TMR-d12 were used for labelling SNAP and Halo, respectively, the latter for expression control and normalization before cells were lysed and subjected to SDS-PAGE. The inventors successfully observed tagged proteins under a fluorescent scanner, both spliced and non-spliced. Encouraged by this, the inventors performed a glycine linker deletion screen (i.e. G95, G96, G232, G270 and combinations thereof) to find mutant CLUSTERₚ₂₇₇ working binary, since SiR-signals were only detectable at the lower molecular weight band. Molecular dynamics simulations and melting points support the higher dynamic nature versus SNAP, and an AlphaFold model of CLUSTERₚ₂₇₇-Halo reveals separation between the split inteins. To gain a deeper insight into the mechanism, the inventors performed kinetic measurements by fluorescence polarization on SNAP, SNAP^{N}-gp-41-1^{C}-SNAP^{C} ± IntN-Halo and CLUSTER277-Halo when exposed to BG-TMR-d12. The inventors found that polarization drops upon addition of the splicing partner, and in the case for CLUSTER were able to extract labelling and splicing kinetics. Endpoint quantification includes controls and indicates less structured features of the spliced fragment. Compared to SNAP-TMR-d12 was unable to react with SNAP^{C}. Next, the inventors verified that the *in vitro* observations are translatable to *in cellulo.* As a proof of concept, the inventors subcloned CLUSTER₂₇₇ onto a Halo-tag bearing nuclear localization sequence (NLS) for transfection in HEK293T cells with nuclear localized CLUSTER₂₇₇-Halo-NLS for dual color labelling. Quantification of signal overlap *versus* a SNAP-Halo-NLS control was non-significant and SDS-PAGE revealed spliced fragments only in the CLUSTER-expressing cells.

To broaden the applicability of CLUSTER, the inventors labelled membrane-bound CLUSTER₂₇₇-TM-Halo and SNAP-TM-Halo (TM=transmembrane) with BG-SiR-d12/CA-TMR-d12. After confocal imaging, SDS PAGE allowed detection of spliced products of different CLUSTER constructs in both channels. Moving to challenging intrinsically disordered proteins, the inventors subcloned CLUSTER from p277 onto the Tau protein, which adopts an ensemble of different conformations²⁷. Transfection in HEK293T cells and Western Blotting (Fig.2C) of TauCLUSTER₂₇₇ and TauSNAP confirmed almost quantitative splicing of TauCLUSTER₂₆₂ revealed comparable expression levels by confocal microscopy. Moreover, both Western blotting and fluorescent SDS-page scanning confirmed almost quantitative splicing of TauCLUSTER₂₆₂. To determine the spliced and non-spliced populations of Tau fusions in live cells and to report on conformational differences, fluorescence lifetime imaging (FLIM) was performed. Indeed, TauCLUSTER fusions differed with significantly longer lifetimes *τ*= 4.1 ns compared to TauSNAP *τ*= 3.8 ns. Overall, the inventors show that CLUSTER can be superiorly used for labeling challenging protein systems in living cells, with different dyes leveraging its small size.

Elegantly, the CLUSTER cassette adds a small, fluorescent label at virtually any position with its strictly stoichiometric reaction. Moreover, the exogenous fluorophore application allows exceptional temporal control. After splicing, the size (~5.2 kDa + cargo) of the CLUSTER systems the product is substantially smaller than the Halo- (33 kDa), or SNAP-tag (20 kDa) or FPs (27 kDa).⁷ Yet bigger than unnatural amino acids,¹¹ the present method and system (CLUSTER), however, overcomes other challenges in terms of costs, toxicity, and putative off-target incorporation.¹² In contrast, other tagging methods (e.g., Lap-tag/LpIA^{W37V} ref¹³ or TC-tag/ReAsH¹⁴) offer restricted cargos or smaller tagging methods (e.g., smaller SpyTag, miniVIPER ^{28,29}) use only peptidic sets for cargo. CLUSTER performs in state-of-the-art imaging approaches, and as such, the inventors anticipate wide adoption of this method across scales in biomedical and life science applications.

### REFERENCES

1. Xue, L., Karpenko, I. A., Hiblot, J. & Johnsson, K. Imaging and manipulating proteins in live cells through covalent labeling. Nat. Chem. Biol. 11, 917-23 (2015).
2. Sahl, S. J., Hell, S. W. & Jakobs, S. Fluorescence nanoscopy in cell biology. Nat. Rev. Mol. Cell Biol. 18, 685-701 (2017).
3. Wolf, P., Gavins, G., Beck-Sickinger, A. G. & Seitz, O. Strategies for Site-Specific Labeling of Receptor Proteins on the Surfaces of Living Cells by Using Genetically Encoded Peptide Tags. Chembiochem 22, 1717-1732 (2021).
4. Terai, T. & Nagano, T. Small-molecule fluorophores and fluorescent probes for bioimaging. Pflugers Arch 465, 347-359 (2013).
5. Thiel, Z., Nguyen, J. & Rivera-Fuentes, P. Genetically Encoded Activators of Small Molecules for Imaging and Drug Delivery. Angew Chem Int Ed Engl 59, 7669-7677 (2020).
6. Wilhelm, J. et al. Kinetic and Structural Characterization of the Self-Labeling Protein Tags HaloTag7, SNAP-tag, and CLIP-tag. Biochemistry 60, 2560-2575 (2021).
7. Erdmann, R. S. et al. Labeling Strategies Matter for Super-Resolution Microscopy: A Comparison between HaloTags and SNAP-tags. Cell Chemical Biology 26, 584-592.e6 (2019).
8. Brunk, E., Mollwitz, B. & Rothlisberger, U. Mechanism to trigger unfolding in O(6) - alkylguanine-DNA alkyltransferase. Chembiochem 14, 703-710 (2013).
9. Mollwitz, B. et al. Directed evolution of the suicide protein O(6)-alkylguanine-DNA alkyltransferase for increased reactivity results in an alkylated protein with exceptional stability. Biochemistry 51, 986-94 (2012).
10. Jumper, J. et al. Highly accurate protein structure prediction with AlphaFold. Nature 596, 583-589 (2021).
11. Elia, N. Using unnatural amino acids to selectively label proteins for cellular imaging: a cell biologist viewpoint. FEBS J 288, 1107-1117 (2021).
12. Zhou, W., Wesalo, J. S., Liu, J. & Deiters, A. Genetic code expansion in mammalian cells: A plasmid system comparison. Bioorg Med Chem 28, 115772 (2020).
13. Uttamapinant, C. et al. A fluorophore ligase for site-specific protein labeling inside living cells. Proc Natl Acad Sci U S A 107, 10914-10919 (2010).
14. Adams, S. R. et al. New biarsenical ligands and tetracysteine motifs for protein labeling in vitro and in vivo: synthesis and biological applications. J Am Chem Soc 124, 6063-6076 (2002).
15. Hirano, M. et al. A highly photostable and bright green fluorescent protein. Nat Biotechnol 40, 1132-1142 (2022).0
16. Liu, T. et al. Gentle Rhodamines for Live-Cell Fluorescence Microscopy. ACS Cent. Sci. 10, 1933-1944 (2024).
17. Grimm, J. B. et al. Optimized Red-Absorbing Dyes for Imaging and Sensing. J. Am. Chem. Soc. 145, 23000-23013 (2023).
18. Dave, K., Gelman, H., Thu, C. T. H., Guin, D. & Gruebele, M. The Effect of Fluorescent Protein Tags on Phosphoglycerate Kinase Stability Is Nonadditive. J. Phys. Chem. B 120, 2878-2885 (2016).
19. Heo, M. et al. Impact of fluorescent protein fusions on the bacterial flagellar motor. Sci Rep 7, 12583 (2017).
20. Pandey, N. K. et al. Fluorescent protein tagging promotes phase separation and alters the aggregation pathway of huntingtin exon-1. Journal of Biological Chemistry 300, 105585 (2024).
21. Ruan, J., Yin, Z. & Yi, P. Effects of fluorescent tags and activity status on the membrane localization of ROP GTPases. Plant Signaling & Behavior 19, 2306790 (2024).
22. Fatti, E., Khawaja, S. & Weis, K. The dark side of fluorescent protein tagging-the impact of protein tags on biomolecular condensation. MBoC 36, br10 (2025).
23. Keppler, A. et al. A general method for the covalent labeling of fusion proteins with small molecules in vivo. Nat Biotechnol 21, 86-9 (2003).
24. Juillerat, A. et al. Engineering substrate specificity of O6-alkylguanine-DNA alkyltransfer-ase for specific protein labeling in living cells. Chembiochem 6, 1263-1269 (2005).
25. Carvajal-Vallejos, P., Pallissé, R., Mootz, H. D. & Schmidt, S. R. Unprecedented Rates and Efficiencies Revealed for New Natural Split Inteins from Metagenomic Sources. Jour-nal of Biological Chemistry 287, 28686-28696 (2012).
26. Roßmann, K. et al. N-Methyl deuterated rhodamines for protein labelling in sensitive fluorescence microscopy. Chem Sci 13, 8605-8617 (2022).
27. Fung, H. Y. J., McKibben, K. M., Ramirez, J., Gupta, K. & Rhoades, E. Structural Characterization of Tau in Fuzzy Tau:Tubulin Complexes. Structure 28, 378-384.e4 (2020).
28. Zakeri, B. et al. Peptide tag forming a rapid covalent bond to a protein, through engineering a bacterial adhesin. Proc. Natl. Acad. Sci. U.S.A. 109, (2012).
29. Doh, J. K., Tobin, S. J. & Beatty, K. E. MiniVIPER Is a Peptide Tag for Imaging and Translocating Proteins in Cells. Biochemistry 59, 3051-3059 (2020).

## Claims

1. A self-labelling bio-orthogonal system comprising:
a. a protein of interest (POI),
b. two autocatalytically self-splicing amino acid sequences, at least one of said sequences being fused to the POI, wherein the self-splicing of the amino acid sequences is inhibited through steric hindrance, induced by
c. an enzyme located between the two autocatalytically self-splicing amino acid sequences, wherein the enzyme comprises a reactive amino acid (aa_{R}), and is capable of unfolding upon a bio-orthogonal reaction of the reactive amino acid (aa_{R}) with a labeled substrate.

2. The system according to claim 1,
wherein the bio-orthogonal reaction of the reactive amino acid (aa_{R}) with a labeled substrate induces (i) the labelling of the aa_{R} (aa_{L}) and (ii) the unfolding of the enzyme (c.), thereby resolving the steric hindrance and inhibition of the two self-splicing amino acid sequences (b.), and inducing an autocatalytic self-splicing reaction of the system.

3. The system according to claim 2, wherein the induced autocatalytic self-splicing reaction results in:
A. a product comprising the POI and the labeled amino acid (aa_{L}) of the enzyme, and
B. a product comprising at least a portion of the two self-splicing amino acid sequences and a remaining portion of the enzyme.

4. The system according to any one of the preceding claims, wherein the two autocatalytically self-splicing amino acid sequences are split intein sequences (In^{N} and Int^{C}), which are preferably inhibited through steric hindrance of the Int^{C}, and/or the In^{N} portion.

5. The system according to any one of the preceding claims, wherein the reactive amino acid (aa_{R}) is a cysteine, preferably wherein the enzyme (c.) comprising the aa_{R} is the *O*⁶-alkylguanine DNA alkyltransferase SNAP, or a derivative or fragment thereof, and the aa_{R} is Cys145 of SNAP.

6. The system according to any one of the preceding claims, wherein labeled-substrate is selected from benzylguanine (BG), chloropyrimidine (CP), chloroalkane (CA), fluorinated labels, pyrimidine-based, and/or rhodamine-based substrates, or derivatives thereof, comprising a label, preferably a fluorescent or radioactive label or dye.

7. The system according to any one of the preceding claims, wherein the autocatalytic self-splicing reaction of the two amino acid sequences is inhibited through steric hindrance induced by circular permutation of the enzyme located between the two autocatalytically self-splicing amino acid sequences.

8. The system according to any one of claims 3-7, wherein the POI (a.) is divided into a C-terminal and an N-terminal portion, and each portion is fused to one of the two autocatalytically self-splicing amino acid sequences (b.) respectively, and wherein the product (A.) resulting from the autocatalytic self-splicing reaction comprises both the C- and N-terminal portions of the POI.

9. The system according to any one of the preceding claims comprising an amino acid sequence selected from SEQ ID NO. 1-32 or a sequence with at least 70% sequence identity thereto.

10. A method of labelling a protein of interest (POI), comprising
- providing the self-labelling bio-orthogonal system according to any one of claims 1-9,
- contacting the system with a labeled substrate, preferably with benzylguanine (BG) comprising a fluorescent or radioactive label or dye,
- thereby inducing a bio-orthogonal labelling reaction of the reactive amino acid (aa_{R}) with the labeled-substrate resulting in (i) the labeling of the aa_{R} (aa_{L}), and (ii) the unfolding of the enzyme (c.),
- thereby inducing the release of steric hindrance and the inhibition of the self-splicing amino acid sequences (b.), and inducing an autocatalytic self-splicing reaction of the system resulting in:
A. a product comprising the POI and the labeled amino acid (aa_{L}) of the enzyme, and
B. a product comprising at least a portion of the two self-splicing amino acid sequences and a remaining portion of the enzyme.

11. The method according to the preceding claim,
wherein the two autocatalytically self-splicing amino acid sequences (b.) are split intein sequences (Int^{N} and Int^{C}), which are preferably inhibited through steric hindrance of the Int^{C}, and/or the Int^{N} portion, and
wherein the reactive amino acid (aa_{R}) is a cysteine, preferably wherein the enzyme (c.) comprising the aa_{R} is the *O*⁶-alkylguanine DNA alkyltransferase SNAP and the aa_{R} is Cys145 of SNAP.

12. The system according to claim 3 or the method according to claim 10, wherein product A. comprises an amino acid sequence selected from SEQ ID NO. 2, 5, 8, 9, 11, 12, 15, 18, 20, 22 or 24, or a sequence with at least 70% sequence identity thereto, preferably at least 80%, 90%, or 95% identity thereto, and product B. comprises an amino acid sequence selected from SEQ ID NO. 3, or 6, or a sequence with at least 70% sequence identity thereto, preferably at least 80%, 90%, or 95% identity thereto.

13. A vector encoding b. and c. of the system according to any one of claims 1-9, and optionally a multiple cloning site (MCS), enabling the insertion of one or more sequences encoding the POI (a.).

14. The vector according to claim 13, comprising a nucleic acid sequence encoding one or more sequences according to SEQ ID NO. 1-32, or a sequence with at least 70% sequence identity thereto, preferably at least 80%, 90%, or 95% identity thereto.

15. A kit comprising the system according to any one of claims 1-9 or the vector according to claims 13-14, and optionally one or more labeled substrates, preferably benzylguanine (BG) comprising a fluorescent or radioactive label or dye.
